# FASCICULE DE BREVET EUROPEEN

(11) **EP 3 291 792 B1**
(45) Date de publication et mention de la délivrance du brevet: **18.12.2024**
(21) Numéro de dépôt: 16720867.7
(22) Date de dépôt: 09.05.2016
(51) Int. Cl.: A61K 8/365, A61K 8/60, A61Q 19/08

(54) **PROCEDE DE TRAITEMENT DES MATIERES KERATINIQUES A PARTIR DE DERIVES DE C-GLYCOSIDES AMIDES, ACIDES OU ESTER, ET LA COMPOSITION COSMETIQUE LES CONTENANT**
VERFAHREN ZUR BEHANDLUNG VON KERATINMATERIALIEN MIT AMID, SÄURE ODER ESTER-C-GLYKOSID-DERIVATEN UND KOSMETISCHE ZUSAMMENSETZUNG DAMIT
PROCESS FOR TREATING KERATIN MATERIALS USING AMIDE, ACID OR ESTER C-GLYCOSIDE DERIVATIVES, AND COSMETIC COMPOSITION CONTAINING SAME

(30) Priorité: 07.05.2015 FR 1554157
(43) Date de publication de la demande: 14.03.2018
(73) Titulaire: L'OREAL, 75008 Paris (FR)
(72) Inventeur: DALKO, Maria, 78000 Versailles (FR); FRANTZ, Marie-céline, 93601 Aulnay-sous-bois (FR); GUEGUINIAT, Amélie, 93601 Aulnay-Sous-Bois (FR); XU, Jinzhu, 75014 Paris (FR); MAO, Lisheng, Shangai 2000032 (CN)
(74) Mandataire: L'Oreal
(86) Numéro de dépôt international: PCT/EP2016/060299
(87) Numéro de publication internationale: WO 2016/177908

(56) Documents cités:
- WO-A1-2012/023584
- WO-A1-94/27575
- WO-A2-2010/004269
- FR-A1- 2 901 133
- JP-A- 2010 195 687
- US-A- 5 834 510
- US-A1- 2005 002 889
- US-A1- 2005 113 313
- FRANK SCHWEIZER ET AL: "Chain Extension of Sugar [delta]-Lactones with the Enolate of tert- Butyl Bromoacetate and Elaboration into Functionalized C -Ketosides, C -Glycosides, and C -Glucosyl Glycines", ORGANIC LETTERS, vol. 3, no. 25, 20 November 2001 (2001-11-20), US, pages 4115 - 4118, XP055210025, ISSN: 1523-7060, DOI: 10.1021/ol0102343
- BRAR A ET AL: "Synthesis of chiral non-proteinogenic 4,5-dihydroxytetrahydropyran derived alpha-amino acids from d-mannitol", TETRAHEDRON LETTERS, PERGAMON, GB, vol. 47, no. 51, 18 December 2006 (2006-12-18), pages 9035 - 9038, XP025005177, ISSN: 0040-4039, [retrieved on 20061218], DOI: 10.1016/J.TETLET.2006.10.099
- DATABASE pubchem [online] 17 March 2015 (2015-03-17), XP055278787, Database accession no. CID 91359529
- YANG ET AL: "Studies on the synthesis of di- and trisaccharide analogues of moenomycin A. Modifications in unit E and in the lipid part", HELVETICA CHIMICA ACTA, VERLAG HELVETICA CHIMICA ACTA, CH, 1 January 2004 (2004-01-01), XP002404113, ISSN: 0018-019X
- PALASZ ALEKSANDRA ET AL: "Application of 2,4,6-trioxo-pyrimidin-5-ylidene alditols in the synthesis of pyrano[2,3-d]pyrimidines containing a sugar moiety by hetero-Diels-Alder reactions and by conjugate Michael addition-cycl", TETRAHEDRON, vol. 69, no. 38, 16 July 2013 (2013-07-16), pages 8216 - 8227, XP028688565, ISSN: 0040-4020, DOI: 10.1016/J.TET.2013.07.032
- "Handbook of Green Chemistry", 1 January 2010, WILEY-VCH VERLAG GMBH & CO. KGAA, Weinheim, Germany, ISBN: 978-3-527-62869-8, article MARIE-CHRISTINE SCHERRMANN ET AL: "Functionalization of Carbohydrates in Water", XP055311633, DOI: 10.1002/9783527628698.hgc056
- P. M. GANNETT ET AL: "In Vitro Reaction of Barbiturates with Formaldehyde", JOURNAL OF ANALYTICAL TOXICOLOGY., vol. 25, no. 6, 1 September 2001 (2001-09-01), US, pages 443 - 449, XP055311675, ISSN: 0146-4760, DOI: 10.1093/jat/25.6.443
- CARPENTER CHRISSIE A ET AL: "Modifications in the nitric acid oxidation ofd-mannose: X-ray crystal structure ofN,N'-dimethyld-mannara", CARBOHYDRATE RESEARCH, PERGAMON, GB, vol. 376, 16 May 2013 (2013-05-16), pages 29 - 36, XP028571342, ISSN: 0008-6215, DOI: 10.1016/J.CARRES.2013.05.004

## Description

L'invention se rapporte à de nouveaux composés C-glycosides à groupe amide, ou acide, une composition cosmétique les comprenant, un procédé de préparation, l'utilisation desdits C-glycosides pour le traitement des matières kératiniques et notamment la peau ; et un procédé de traitement des matières kératiniques mettant en oeuvre lesdits C-glycosides.

Particulièrement lesdits C-glycosides de l'invention sont des inducteurs de l'expression de GLUT-1,

La présente invention se rapporte en particulier au domaine du vieillissement et des signes qui lui sont associés, sur la peau et/ou ses annexes. Elle concerne en particulier la modulation de l'équilibre entre la prolifération et la différenciation des cellules épidermiques et l'amélioration des signes liés au phénomène d'amincissement de l'épiderme dû à une diminution du nombre de kératinocytes en phase de prolifération.

Les femmes - et les hommes - ont tendance actuellement à vouloir paraître jeunes le plus longtemps possible et cherchent par conséquent à estomper les marques du vieillissement de la peau, qui se traduisent notamment par des rides et des ridules, un amincissement de l'épiderme et/ou un aspect de peau molle et flétrie. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

La peau est constituée de deux compartiments, l'un superficiel, l'épiderme, et l'autre plus profond, le derme, qui interagissent. L'épiderme humain naturel est composé principalement de trois types de cellules qui sont les kératinocytes, très majoritaires, les mélanocytes, et les cellules de Langerhans. Chacun de ces types cellulaires contribue par ses fonctions propres au rôle essentiel joué dans l'organisme par la peau, notamment le rôle de protection de l'organisme des agressions extérieures appelé « fonction barrière ».

L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline, et enfin la couche cornée (ou *stratum corneum*), constituée d'un ensemble de couches de kératinocytes au stade terminal de leur différenciation et appelés cornéocytes. Les cornéocytes sont des cellules anucléées principalement constituées d'une matière fibreuse contenant des cytokératines, entourée d'une enveloppe cornée.

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses. La cohésion entre l'épiderme et le derme est assurée par la jonction dermo-épidermique.

Il y a en permanence dans l'épiderme production de nouveaux kératinocytes pour compenser la perte en continu de cellules épidermiques au niveau de la couche cornée. Cependant, au cours du vieillissement, on peut observer de façon physiologique une diminution du nombre de cellules en phase de prolifération, et par conséquent une diminution des couches épidermiques vivantes. En limitant et/ou retardant le passage des cellules en phase de différenciation, on maintient le pool de cellules jeunes.

Il est donc important de préserver ce pool de cellules prolifératives, en évitant ou retardant leur différenciation, afin de contribuer à retarder l'apparition des signes du vieillissement.

Le transport du glucose dans la plupart des cellules de mammifères est régulé par une famille de protéines membranaires appelées « glucose transporters », aussi désignées par GLUT. Au moins 13 protéines sont codées par une famille de gènes apparentés, ces protéines présentant différents domaines transmembranaires. L'expression des différentes isoformes de GLUT varie en fonction du tissu et des conditions hormonales et environnementales.

Les kératinocytes expriment au moins 4 types de transporteurs de glucose, GLUT-1, 2, 3 et 5. Dans l'art antérieur, un certain nombre d'applications sont décrites avec des inducteurs de GLUT.

Ainsi, US 2003/0187036 propose l'utilisation de dérivés de biguanides pour favoriser la cicatrisation des lésions cutanées telles que les ulcères survenant chez les diabétiques, ou d'autres blessures.

US 2005/0037440 se rapporte à l'identification de facteurs de longévité chez les mammifères, et au rôle du facteur cJUN et de la modulation du signal JNK. Il suggère ainsi d'utiliser des agents diminuant l'activité d'un indicateur comme DAF-14, la supe-roxyde-dismutase, GLUT-1 ou GLUT-4 pour lutter contre les signes du vieillissement causés principalement par le stress oxydatif.

DE 10259966 décrit des compositions cosmétiques ou pharmaceutiques contenant des acides aminés de type mycosporine et améliorant la capture de l'oxygène; ces composés induisent notamment une réduction de l'expression de GLUT-1. Les compositions peuvent notamment être appliquées pour le traitement du vieillissement de la peau. FR2901133 décrit l'utilisation dans une composition contenant un milieu physiologiquement acceptable d'au moins un composé inducteur de glycolyse épidermique (à l'exclusion de IGF-1) comme agent pour diminuer et/ou retarder les signes du vieillissement de la peau et/ou de ses annexes. Avantageusement, le composé inducteur de glycolyse est un activateur des peptides transporteurs du glucose (GLUT).

Cependant, il existe toujours un besoin de trouver de nouveaux agents anti-âge utiles.

Par ailleurs les glycosides sont très intéressants dans des domaines variés dont la cosmétique, néanmoins leur synthèse chimique, notamment pour les C-glycosides ne sont pas toujours faciles et accessibles. En effet il faut généralement soit halogéner l'atome de carbone en position 1 du sucre puis lui faire subir des réactions de substitution par alkylation, organomagnésiens, organolithiés, etc, en ayant au préalable pris le soin de protéger les groupes hydroxy ou amino du sucre puis de les déprotéger ensuite (voir par ex. « recent advances in stereoselective C-glycoside synthesis », tetrahedron, 54, 9913-9959 (1998)). Une autre solution est d'utiliser une glycosylation par catalyse aux métaux de transition. Cependant leur mise en oeuvre n'est pas toujours aisée et en plus nécessite l'emploi de catalyseurs métalliques et de ligands coûteux, non nécessairement industriels, et devant être compatibles avec l'utilisation cosmétique des C-glycosides synthétisés (voir par ex. « recent advances in transition métal catalyzed glycosylation» ACS catal,, 3, 2(8), 1563-1595 (2012)). En outre, ces voies de synthèses ne permettent pas d'obtenir à partir d'un même réactif des amides, esters ou acides de sucres sans avoir à utiliser la protection et déprotection des groupes hydroxy ou amino. De plus la plupart des réactions pour obtenir des C-glycosides sont réalisées dans des solvants qui ne sont pas toujours écocompatibles ou difficile d'usage en industrie. Il est donc souhaitable autant que possible d'utiliser des solvants cométiquement acceptables, écocompatibles et en particulier l'eau.

Par ailleurs, au cours du vieillissement chronologique et/ou actinique, le derme et l'épiderme subissent de nombreuses modifications et dégradations qui se traduisent, avec l'âge, par une flaccidité et une perte de souplesse cutanée.

Parmi les éléments dégradés (notamment collagène et élastine), les protéoglycanes (appelés aussi PGs) et les glycosaminoglycanes (appelés aussi GAGs) sont également altérés. En effet, au cours du vieillissement, les fibroblastes et les kératinocytes produisent de moins en moins de PGs et de GAGs et leur synthèse est imparfaite. Il en résuite une désorganisation importante : le dépôt des GAGs sur le squelette protéique formant le PG est anormal, ce qui a pour conséquence une diminution de de la tonicité des tissus et donc et de perte de souplesse de la peau.

Les composés (I) , et en particulier les composés (Ic) décrits ci-après, utilisés selon l'invention présentent également une bonne activité de desquamation de la peau.

La Demanderesse a mis en évidence de façon surprenante que certains composés C-glycosides à groupe amide ou ester ou acide ont des propriétés anti-âge.

En outre la demanderesse a trouvé un moyen de synthèse facile pour accéder à une grande variété de C-glycosides notamment par l'emploi de dérivé d'acide barbiturique puis d'agent oxydant tel que le peroxyde d'hydrogène ou système générateur de peroxyde d'hydrogène. Cette méthode est compatible avec des motifs sucres aux fonctions hydroxy et éventuellement amino non protégées.

La présente invention a donc pour objet l'utilisation cosmétique d'un composé C-glycoside de formule (I) telle que définie ci-après comme agent anti-âge.

L'invention a également pour objet des nouveaux composés de formule (I) tels que définis ci-après.

Les composés **(I)** et **(I‴)** permettent de traiter les matières kératiniques et notamment la peau en particulier pour diminuer et/ou retarder les signes du vieillissement de la peau et/ou de ses annexes.

L'invention concerne également un procédé de traitement cosmétique des matières kératiniques en particulier de la peau, comprenant l'application sur lesdites matières, comprenant une composition cosmétique comprenant au moins un composé de formule **(I)** ou **(I‴).**

Par « *peau* », on entend la peau du visage et/ou du corps, le cuir chevelu.

Par « *annexes* », on entend les cils, les sourcils, les ongles, et les cheveux, en particulier les cils et les cheveux.

Selon un autre mode particulier de l'invention, la composition est destinée à l'administration topique sur les matières kératiniques telles que la peau.

Les composés **(I)** et **(I‴)** permettent notamment de prévenir et/ou traiter les signes du vieillissement cutanés.

Parmi les signes cutanés du vieillissement, on cite notamment une perte de fermeté et/ou d'élasticité et/ou de tonicité et/ou de souplesse de la peau, la formation des rides et des ridules, les rides d'expression, en particulier au niveau du front et de l'espace intersourcillier, les rides et/ou ridules péri-buccales, et/ou le relâchement au niveau du contour des lèvres, en particulier au niveau de la lèvre blanche (zone située entre la lèvre supérieure et le nez), un aspect terne du teint, l'aspect papyracé de la peau.

Les composés (I) et (I‴) présentent avantageusement une activité de stimulation de la synthèse des glycosaminoglycanes et permettent ainsi de prévenir et/ou de traiter les signes de vieillissement cutané, en particulier la perte de fermeté et/ou d'élasticité et/ou de tonicité et/ou de souplesse de la peau.

Un premier objet de l'invention est un procédé de traitement des matières kératiniques, mettant en oeuvre au moins une composition cosmétique comprenant au moins un composé de formule **(I)** suivante : 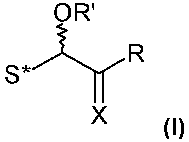 ainsi que leurs solvates tels que les hydrates, leurs isomères optiques, géométriques, tautomères et leurs sels de base ou d'acide, organiques ou minéraux, formule **(I)** dans laquelle :
- **S*** désigne un monosaccharide choisi parmi le D-glucose, le D-xylose, le L-rhamnose, le D-mannose, le D-galactose ou un disaccharide choisi parmi D-lactose, D-maltose et D-cellobiose ;
   ledit radical mono ou disaccharide étant relié au reste de la molécule par une liaison entre l'atome de carbone C₁ d'un des sucres dudit radical mono ou polysaccharide, cette liaison pouvant être anomérique α ou β ;
- **X** représente un atome d'oxygène ;
- **R'** représente un atome d'hydrogène ;
- **R** représente un groupe choisi parmi : i) hydroxy ; et ii) amino - NR₁R₂ avec R₁ et R₂, identiques ou différents, représentant un atome d'hydrogène, un groupe (C₁-C₁₈)alkyle; de préférence alkyle en C₁-C₁₆ tel que méthyle, n-propyle, n-hexyle, n-tétradécyle (n-C₁₄).

Un autre objet de l'invention est l'utilisation des composés de formule **(I)** tels que définis précédemment et préférentiellement l'utilisation des composés de formule **(I‴)** pour le traitement cosmétique des matières kératiniques 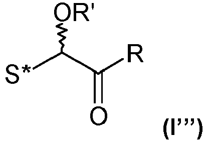 ainsi que leurs solvates tels que les hydrates, leurs isomères optiques, géométriques, tautomères et leurs sels de base ou d'acide, organiques ou minéraux,

Formule **(I‴)** dans laquelle **S*,** et **R** sont tels que définis précédemment et **R'** désigne un atome d'hydrogène.

Au sens de la présente invention, et à moins qu'une indication différente ne soit donnée :
- les cycles saturés ou insaturés, éventuellement condensés, peuvent être aussi éventuellement substitués ;
- les radicaux « *alkyle* » sont des radicaux hydrocarbonés, saturés, linéaires ou ramifiés, généralement en C₁-C₁₈, particulièrement en C₁-C₁₀, de préférence les radicaux alkyles en C₁-C₆ ; comme groupe alkyle en C₁-C₁₄, on peut notamment citer les groupes méthyle, éthyle, isopropyle, n-propyle, n-butyle, t-butyle, isobutyle, sec-butyle, pentyle, isopentyle, néopentyle, n-hexyle, n-heptyle et octyle ;

Selon un mode de réalisation particulier de l'invention les composés de formule **(I)** sont tels que, pris ensemble ou séparément, **R'** représente un atome d'hydrogène et/ou **R** représente un groupe hydroxy ou amino -N(H)-R', avec R'₁ représentant un groupe alkyle en C₁-C₁₆, de préférence méthyle, n-propyle, n-hexyle, n-tétradécyle et/ou X représente un atome d'oxygène.

Il est entendu que pour les composés de formule **(I)** telle que définie précédemment lorsque **S*** représente un radical monosaccharide alors il peut se trouver sous forme pyranose (l'hétérocycle sucre qui le constitue est à 6 chainons) ou furanose (l'hétérocycle sucre qui le constitue est à 5 chainons) ; et lorsque **S*** représente un radical disaccharide il comprend l'enchainement de 2 saccharidiques identiques ou différentes entres elles qui peuvent être sous forme furanose, ou pyranose. De préférence le disaccharide qui résulte de l'enchainement d'une unité saccharidique sous forme furanose et d'une unité sous forme pyranose ou l'enchainement d'une unité saccharidique sous forme pyranose et d'une unité sous forme furanose ; que ce soit pour le radical monosaccharide ou polysaccharidique chaque unité saccharidique pouvant se trouver sous forme L lévogyre ou D dextrogyre, et sous forme anomérique α ou β.

En particulier, **S*** représente un monosaccharide choisi parmi le D-glucose, le D-xylose, le L-xylose, et le L-rhamnose.

De préférence, **S*** représente un sucre choisi parmi le glucose, le xylose et le lactose. Préférentiellement, **S*** désigne le glucose ou le xylose. En particulier, **S*** désigne le D-glucose ou le D-xylose.

Plus préférentiellement, **S*** désignent le glucose. En particulier, **S*** désigne le D-glucose.

Selon un mode de réalisation particulier le radical **S*** représente un disaccharide choisi parmi le D-lactose, le D-cellobiose, le D-maltose.

Selon un mode de réalisation particulier **R** représente de préférence un groupe N(H)R₁ et plus particulièrement N(H)CH₃.

Selon un mode de réalisation particulier de l'invention les composés de formule **(I)** sont tels que **R** représente un groupe amino - NR₁R₂ avec R₁ et R₂, identiques ou différents, représentent un atome d'hydrogène, (C₁-C₁₈)alkyle, linéaire ou ramifié; de préférence alkyle en C₁-C₁₆ tel que méthyle, n-propyle, n-hexyle, n-tétradécyle (n-C₁₄).

Selon un mode de réalisation de l'invention, les composés **(I)** sont des composés de formule **(Ia)** (composés amide) : 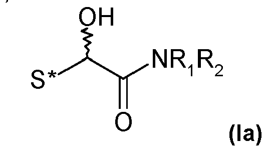 ainsi que leurs solvates tels que les hydrates, leurs isomères optiques, géométriques, tautomères et leurs sels de bases ou d'acide, organiques ou minéraux, Formule **(Ia)** dans laquelle **S***, **R₁,** et **R₂** sont tels que définis précédemment.

De préférence, **S*** désigne un sucre choisi parmi le D-glucose, le D-xylose, le L-rhamnose, le D-mannose, le D-galactose, le D-lactose.

De préférence, **R₁** représente un atome d'hydrogène ; **R₂** représente un radical alkyle en C₁-C₁₈, linéaire ou ramifié, saturé ou insaturé, de préférence en C₁-C₁₆ tel que méthyle, n-propyle, n-hexyle, n-tétradécyle.

Plus préférentiellement, **R₁** représente un atome d'hydrogène ; **R₂** représente un radical alkyle en C₁-C₅, linéaire ou ramifié, tel que méthyle, éthyle, n-propyle, n-butyle, n-pentyle.

Particulièrement **S*** désigne un sucre choisi parmi le glucose, le xylose, le rhamnose, le mannose, le galactose, le lactose ; **R₁** représente un atome d'hydrogène ; **R₂** désigne un radical alkyle en C₁-C₁₈, linéaire ou ramifié ; plus particulièrement R₂ désigne un radical alkyle en C₁-C₁₆, linéaire ou ramifié ; préférentiellement R₂ désigne un radical choisi parmi méthyle, n-propyle, n-hexyle, n- tétradécyle.

Préférentiellement **S*** désigne un sucre choisi parmi le glucose ; **R₁** représente un atome d'hydrogène ; **R₂** désigne un radical alkyle en C₁-C₁₈, linéaire ou ramifié ; plus particulièrement R₂ désigne un radical alkyle en C₁-C₁₆, linéaire ou ramifié ; préférentiellement R₂ désigne un radical choisi parmi méthyle, n-propyle, n-hexyle, tétradécyle.

Pour les composés (la) décrits précédemment, **R₂** représente de préférence un radical méthyle ou propyle et préférentiellement un radical méthyle.

Comme exemple de composés de formule **(Ia),** on peut en particulier citer les composés suivants :

| **Composé** | **S* = D-Glucose** |
|---|---|
| **1** | 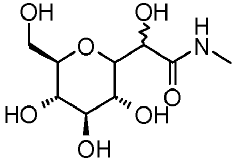 |
| **2** | 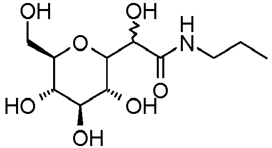 |
| **3** | 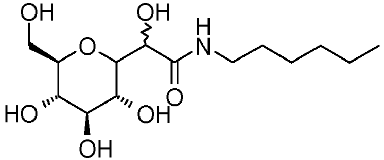 |
| **4** | 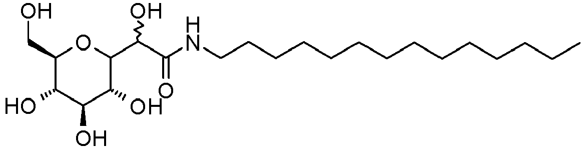 |

| **Composé** | **S* = L-Xylose** |
|---|---|
| **5** | 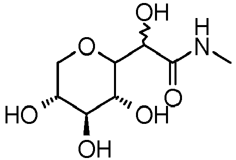 |
| **6** | 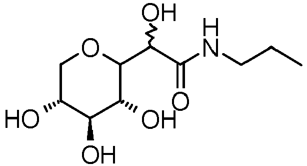 |
| **7** | 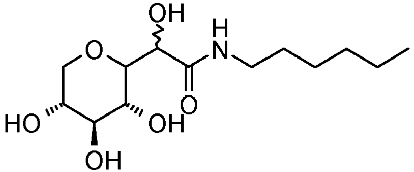 |
| **8** | 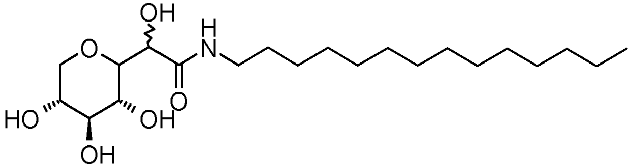 |

| **Composé** | **S* = L-Rhamnose** |
|---|---|
| **9** | 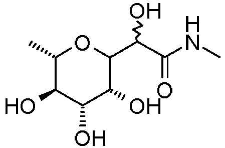 |
| **10** | 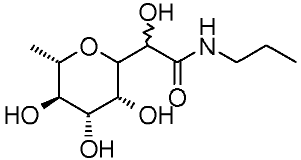 |
| **11** | 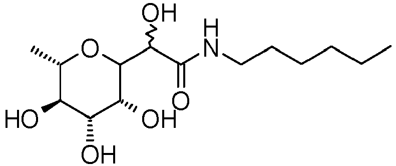 |
| **12** | 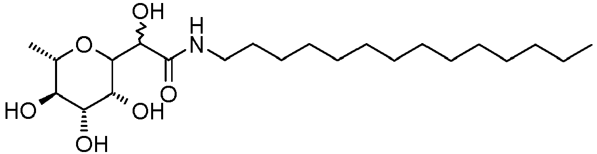 |

| **Composé** | **S* = D-mannose** |
|---|---|
| **13** | 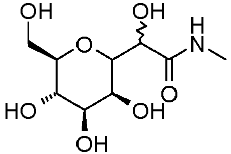 |

| | |
|---|---|
| **14** | 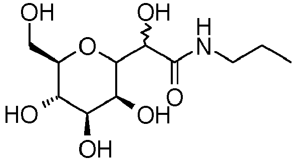 |
| **15** | 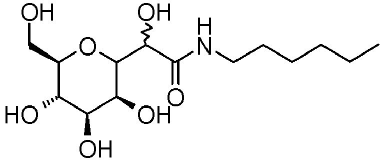 |
| **16** | 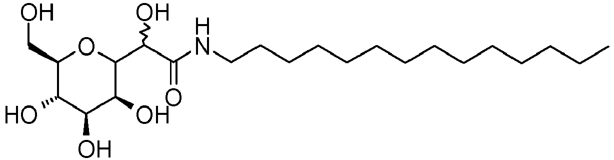 |

| **Composé** | **S* = D-galactose** |
|---|---|
| **17** | 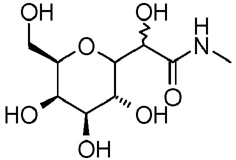 |
| **18** | 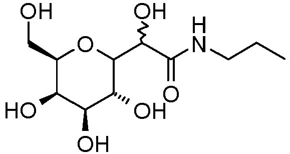 |
| **19** | 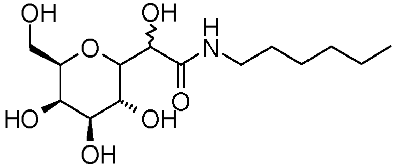 |
| **20** | 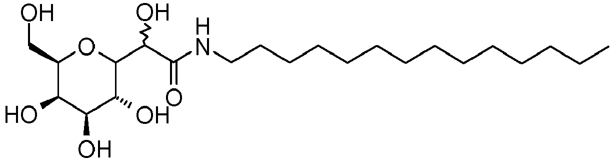 |

| **Composé** | **S* = D-lactose** |
|---|---|
| **21** | 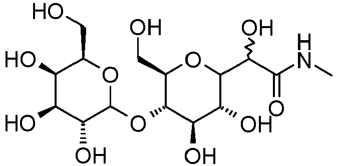 |
| **22** | 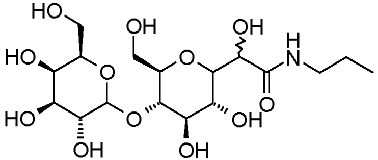 |
| **23** | 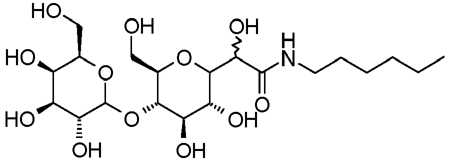 |
| **24** | 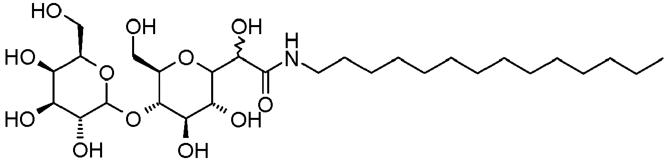 |

ainsi que leurs solvates tels que les hydrates, et leurs sels de base ou d'acide, organiques ou minéraux.

Les composés 1, 2, 5, 6, 9, 10, 13, 14, 17, 18, 21, 22 décrits précédemment sont particulièrement préférés.

Selon un deuxième mode de réalisation selon l'invention, les composés **(I)** sont des composés de formule **(Ic)** (composés acide : 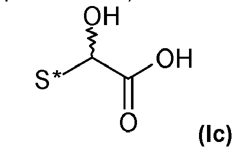 ainsi que leurs solvates tels que les hydrates, leurs isomères optiques, géométriques, tautomères et leurs sels de bases ou d'acide, organiques ou minéraux, Compose dé formule **(Ic)** dans laquelle **S*** est tel que défini précédemment. Comme composé **(Ic)** ont peut citer les composés suivants :

| **Composé** | **S* = D glucose** |
|---|---|
| **4** | 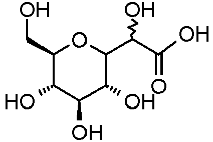 |
| **9** | |
| | **S* = D galactose** |
| **50** | 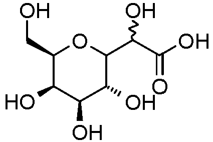 |
| | **S* = D mannose** |
| **51** | 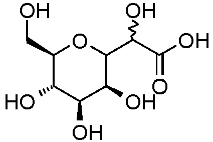 |
| | **S* = D xylose** |
| **52** | 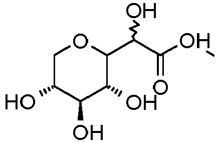 |
| | **S* = L rhamnose** |
| **53** | 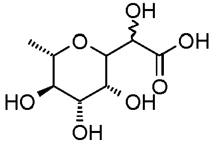 |

| **Composé** | **S* = D-lactose** |
|---|---|
| **54** | 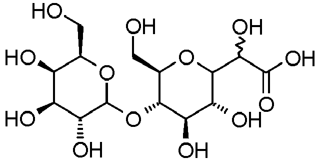 |

ainsi que leurs solvates tels que les hydrates, et leurs sels de base ou d'acide, organiques ou minéraux,

Les solvates acceptables des composés utilisés dans la présente invention comprennent des solvates conventionnels tels que ceux formés lors de la dernière étape de préparation desdits composés du fait de la présence de solvants. A titre d'exemple, on peut citer les solvates dus à la présence d'eau (hydrates) ou d'alcools linéaires ou ramifiés comme l'éthanol ou l'isopropanol.

Les sels des composés **(I)** qui comprennent au moins une fonction acide peuvent être choisis parmi les sels métalliques, par exemple aluminium (Al³⁺), zinc (Zn²⁺), manganèse (Mn²⁺) ou cuivre (Cu²⁺), les sels alcalins, par exemple lithium (Li⁺), sodium (Na⁺) ou potassium (K⁺), ou alcalino-terreux, par exemple calcium (Ca²⁺) ou magnésium (Mg²⁺). Il peut également s'agir de dérivés ammonium de formule NH₄⁺ ou de sels organiques tels que des ammoniums de formule Y₃NH⁺, NY₃ désignant une amine organique, les radicaux Y étant identiques ou différents, deux ou trois radicaux Y pouvant former deux à deux un cycle avec l'atome d'azote qui les porte ou NY₃ pouvant désigner une amine aromatique. Les amines organiques sont par exemple des alkylamines, comme par exemple la méthylamine, la diméthylamine, la triméthylamine, la triéthylamine ou l'éthylamine, ou des hydroxyalkylamines, comme par exemple la 2-hydroxyéthylamine, la bis-(2-hydroxyéthyl)amine ou la tri-(2-hydroxyéthyl)amine, ou des cycloalkylamines, comme par exemple la bicyclohexylamine ou la glucamine, la pipéridine, ou des pyridines et analogues, par exemple la collidine, la quinine ou la quinoline, ou des acides aminés à caractère basique, comme par exemple la lysine ou l'arginine ;
Les sels des composés de formule (I) qui comprennent au moins un fonction amine peuvent également être salifiés par un acide organique tel que l'acide citrique, acide lactique, acide tartrique, acide aspartique, acide glutamique, acide acétique, acide formique, acide trifluoroacétique, acide chlorhydrique, acide glycolique, ou l'acide malique.

Dans le cas où les composés selon l'invention sont sous forme de sel, les cations se trouvent bien entendu en une quantité assurant l'électroneutralité des composés de formule (I).

Les sels minéraux des composés de formule (I) selon l'invention peuvent être avantageusement choisis parmi les sels métalliques Cu²⁺, Mn²⁺ et Zn²⁺, les sels alcalins Li⁺, Na⁺ et K⁺ et les sels alcalino-terreux Ca²⁺ et Mg²⁺.

Selon une autre variante, les sels organiques des composés de formule (I) selon l'invention peuvent être avantageusement choisis parmi les ammoniums, de préférence parmi les sels d'acides aminés à caractère basique comme par exemple la lysine ou l'arginine ou parmi les sels de diéthanolamine ou de triéthanolamine.

De préférence, les composés **(I)** sont sous forme de sels de sodium Na⁺.

De préférence, les composés **(I)** sont sous forme de sels de potassium K⁺.

De préférence, les composés **(I)** sont sous forme de sels de calcium Ca²⁺.

L'invention a également pour objet les composés nouveaux de formule (I) pour lesquels :
- **S*** représente un radical sucre monosaccharide choisi parmi : le glucose, le galactose, le mannose, le xylose, le rhamnose, ou un disaccharide choisi parmi : le lactose, le maltulose;
   ledit radical **S*** comprenant un ou plusieurs radicaux **-ORₛ** et éventuellement un ou deux radicaux -NHR'ₛ,
   ledit radical mono ou disaccharide étant relié au reste de la molécule par une liaison entre l'atome de carbone C¹ du sucre ou d'un des sucres et cette liaison pouvant être anomérique α ou β;
- **X** représente un atome d'oxygène ou de soufre de préférence oxygène ;
- **R'** représente :
   i) un atome d'hydrogène ;
- **R** représente un groupe choisi parmi :
   i) hydroxy ;
   iv) amino NR₁R₂ avec R₁ et R₂ désignant indépendamment un atome d'hydrogène, un radical (C₁-C₁₆)alkyle tel que méthyle, n-propyle, n-hexyle, n-tetradecyle;
- **Rₛ** représente un atome d'hydrogène
- lorsque **S*** représente un mannose, **R'** représente un atome d'hydrogène, alors **R** est différent d'un groupe diéthylamino N(Et)₂.

Comme composés nouveaux (I) on peut citer les composés 1 à 24 et 49 à 54 décrits précédemment.

De préférence les composés nouveaux sont les composés (I) nouveaux pour lesquels R = -NR1R2 tel que défini précédemment.

Les composés nouveaux préférés sont les composés 1 à 24 décrits précédemment. Les composés nouveaux plus préférés sont les composés 1, 2, 5, 6, 9, 10, 13, 14, 17, 18, 21, 22 décrits précédemment.

La présente invention concerne également une composition comprenant, dans un milieu physiologiquement acceptable, un composé de formule (I) tel que décrit ci-dessus. La composition est en particulier une composition cosmétique.

Selon une forme avantageuse de l'invention, la composition cosmétique renferme au moins un composé de formule (I) tel que défini précédemment et au moins un additif choisi parmi un parfum, un agent épaississant, un tensio-actif, un pigment, un colorant, un agent conservateur.

Plus particulièrement, la composition cosmétique selon l'invention renferme au moins un composé choisi parmi les composés 1 à 24 et 49 à 54 définis précédemment, ainsi que leurs solvates tels que les hydrates, et leurs sels de base ou d'acide, organiques ou minéraux,

Le composé de formule (I) (chaque composé de formule (I) si la composition en comprend plusieurs) peut être présent dans la composition, en une quantité qui peut être comprise entre 0,01 et 10% en poids, de préférence entre 0,1 à 5% en poids, notamment entre 0,5 à 3% en poids, par rapport au poids total de la composition.

L'invention a également pour objet un procédé de traitement cosmétique de la peau, comprenant l'application sur la peau, d'une composition comprenant au moins un composé de formule (I) telle que définie précédemment.

En particulier, le procédé de traitement vise à prévenir les signes du vieillissement cutané.

De préférence, le procédé de traitement vise à traiter les signes du vieillissement cutané.

Elle porte encore sur un procédé de traitement cosmétique de la peau destiné à prévenir et/ou traiter le vieillissement comprenant au moins une étape consistant à appliquer sur une peau présentant des signes de vieillissement cutané au moins une composition comprenant composé de formule (I) telle que définie précédemment.

En particulier, le procédé selon l'invention vise à améliorer l'éclat et/ou l'uniformité du teint ; améliorer la radiance et/ou la transparence de la peau ; améliorer la douceur, la souplesse et/ou l'élasticité de la peau ; et/ou prévenir et/ou diminuer les rides et/ou ridules.

Les composés de formule (I) sont notamment utiles comme agents pour lutter contre les signes du vieillissement, en particulier du vieillissement chronobiologique, de la peau

La présente invention concerne également l'utilisation d'au moins un composé de formule (I), un de ses sels ou un de leurs solvates, pour la préparation d'une composition destinée à lutter contre les signes du vieillissement, en particulier du vieillissement chronobiologique, de la peau. Elle concerne également l'utilisation d'au moins un composé de formule (I) tel que défini ci-avant dans une composition cosmétique comprenant un milieu physiologiquement acceptable, en tant qu'agent destiné à lutter contre les signes du vieillissement de la peau.

La présente invention concerne également l'utilisation d'un composé ou d'une composition selon l'invention pour lutter contre les signes du vieillissement, en particulier du vieillissement chronobiologique, de la peau.

Les composés ou compositions selon l'invention sont notamment destinés à la correction de tous les désordres de ré-épithélialisation de la peau.

A ce titre, on utilisera en particulier les composés de formule (I) tels que :
- **S*** représente un radical sucre monosaccharide choisi parmi : le glucose, le galactose, le mannose, le xylose, le rhamnose,; ou un disaccharide choisi parmi : le lactose, le cellobiose, le maltose;
   ledit radical mono ou disaccharide étant relié au reste de la molécule par une liaison entre l'atome de carbone C¹ du sucre ou d'un des sucres et cette liaison pouvant être anomérique α ou β;
- **X** représente un atome d'oxygène ;
- **R'** représente :
   i) un atome d'hydrogène ;
- **R** représente un groupe choisi parmi :
   i) hydroxy ;
   ii) amino NR₁R₂ avec R₁ et R₂ désignant indépendamment un atome d'hydrogène, un radical (C₁-C₁₆)alkyle tel que méthyle, n-propyle, n-hexyle, n-tetradecyle;

Selon autre variante on utilisera en particulier un composé de formule (I) tel que
- **S*** désigne un radical sucre monosaccharide choisi parmi : le glucose, le galactose, le mannose, le xylose, le rhamnose,; ou un disaccharide choisi parmi : le lactose, le cellobiose, le maltose ;
   ledit radical mono ou disaccharide étant relié au reste de la molécule par une liaison entre l'atome de carbone C¹ du sucre ou d'un des sucres et cette liaison pouvant être anomérique α ou β;
- **X** désigne un atome d'oxygène ;
- **R'** désigne
   i) un atome d'hydrogène ;
- **R** désigne un groupe choisi parmi :
   i) hydroxy ;
   ii) amino NR₁R₂ avec R₁ et R₂ désignant indépendamment un atome d'hydrogène, un radical (C₁-C₁₆)alkyle tel que méthyle, n-propyle, n-hexyle, n-tetradecyle;

Plus particulièrement, on utilisera un composé choisi parmi les composés 1 à 24, et 49 à 54 définis précédemment, , ainsi que leurs solvates tels que les hydrates, et leurs sels de base ou d'acide, organiques ou minéraux.

Les composés ou compositions selon l'invention sont particulièrement adaptés pour lutter contre les signes du vieillissement chronobiologique de l'épiderme. Au cours du vieillissement chronobiologique, l'épaisseur de l'épiderme se réduit, les divisions cellulaires diminuant en nombre. En facilitant la multiplication cellulaire, en particulier des cellules de l'épiderme, on facilite sa régénération et la peau a un aspect plus jeune.

Les composés ou compositions selon l'invention sont notamment destinés à prévenir ou diminuer les rides et les ridules, et/ou l'amincissement de la peau et/ou l'aspect de peau molle et/ou flétrie. La présente invention concerne ainsi également l'utilisation d'au moins un composé selon l'invention dans une composition cosmétique pour prévenir ou diminuer les rides et les ridules, et/ou l'amincissement de la peau et/ou l'aspect de peau molle et/ou flétrie.

La composition comprend en outre un milieu physiologiquement acceptable, qui est préférentiellement un milieu cosmétiquement ou pharmaceutiquement, notamment dermatologiquement, acceptable, c'est-à-dire sans odeur, couleur ou aspect désagréable, et qui ne génère pas de picotement, tiraillement ou rougeur inacceptable pour l'utilisateur. En particulier la composition est adaptée à une application topique sur la peau et les annexes.

Par milieu physiologiquement acceptable, on comprend un milieu compatible avec la peau d'êtres humains et les annexes.

La composition selon l'invention peut alors comprendre tous les adjuvants usuellement employés dans le domaine d'application envisagée.

On peut notamment citer l'eau; les solvants organiques, notamment les alcools en C1-C6 et les esters d'acide carboxylique en C₂-C₁₀; les huiles carbonées et/ou siliconées, d'origine minérale, animale et/ou végétale; l'eau, les cires, les pigments, les charges, les colorants, les tensioactifs, les émulsionnants, les co-émulsionnants; les actifs cosmétiques ou dermatologiques, les filtres UV, les polymères, les gélifiants hydrophiles ou lipophiles, les épaississants, les conservateurs, les parfums, les bactéricides, les absorbeurs d'odeur, les antioxydants.

Ces éventuels adjuvants peuvent être présents dans la composition à raison de 0,001 à 80% en poids, notamment 0,1 à 40% en poids, par rapport au poids total de la composition.

La composition selon l'invention peut constituer une composition de maquillage, ou préférentiellement de soin de la peau, et notamment une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage, un composition capillaire.

La composition selon l'invention est avantageusement une composition anti-âge, notamment de soin, destinée à traiter et/ou lutter contre, cosmétiquement, les signes extérieurs du vieillissement cutané; la composition est plus particulièrement une composition de soin des peaux matures.

L'invention est illustrée plus en détail par les exemples non limitatifs suivants.

### EXEMPLES

### Exemple 1 : Synthèse du composé 1

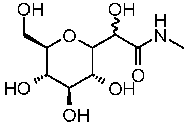

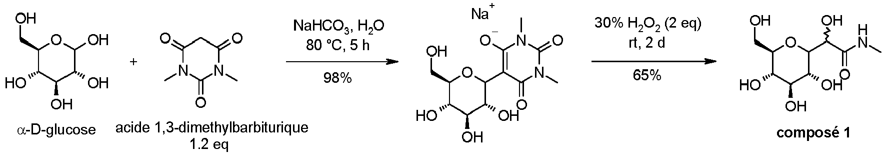

**Etape 1.** Dans un ballon monocol de 250 mL, est introduit le D-glucose (5 g), solubilisé dans l'eau (56 mL). L'acide 1,3-dimethylbarbiturique (4.33 g) est ajouté sous agitation, puis NaHCO₃ est ajouté jusqu'à pH 7. Après neutralisation, le ballon est équipé d'un réfrigérant et le mélange est chauffé à 80 °C pendant 5 h. La réaction est suivie par CCM dans 1:1 DCM/MeOH. Le mélange réactionnel est concentré sous vide. Le résidu obtenu est repris dans l'eau puis précipité dans l'acétone, filtré et le solide obtenu est séché sous vide. Poudre orange, 9.3 g, rdt 98%. Les spectres RMN ¹H et spectrométrie de masse sont en accord avec la structure attendue.

**Etape 2.** 5 g du produit obtenu en étape 1 sont dilués dans 10 mL d'eau à température ambiante, puis 30% H₂O₂ (3 mL) sont ajoutés. Le mélange réactionnel est agité à température ambiante pendant 2 jours. La réaction est suivie par CCM dans BuOH/AcOH/H₂O (3/1/1). Puis sont ajoutés 0.21 equiv. d'une solution aqueuse à 20% de metabisulfite de sodium, et l'agitation est poursuivie pendant 1 h à température ambiante. L'absence de peroxyde est vérifiée, puis le mélange réactionnel est versé dans 300 ml d'éthanol. Le précipité formé est filtré, et le filtrat concentré à un volume minimum. La deuxième fraction de solide est collectée et les deux fractions sont séchées sous vide à 30 °C (1.6 g). Le filtrat est à nouveau repris dans l'éthanol, le précipité formé est filtré, et le filtrat concentré à sec pour donner une autre fraction de produit (0.8 g). Solide blanc, 2.4 g, rendement 65%. Les spectres RMN ¹H / ¹³C et spectrométrie de masse sont en accord avec la structure attendue.

### Exemple 2 : Synthèse du composé 5

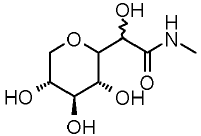

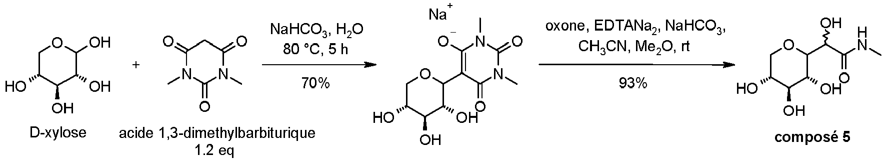

**Etape 1.** Suivant le procédé décrit dans l'exemple 1, l'intermédiaire est obtenu à partir du D-xylose. Rdt 70%.

**Etape 2.** 5 g du produit obtenu en étape 1 sont dilués dans 80 mL d'acétonitrile à t.a. 7 mg (0.0012 eq) d'EDTANa₂ préalablement dilué dans 46 mL d'eau (C = 4.10⁻⁴ M) sont ajoutés. Le mélange est placé dans un bain de glace puis 13 mL d'acétone y sont versés. Le mélange Oxone (25.2 g, 2.5 eq) / NaHCO₃ (10.5 g, 8 eq) est ajouté lentement au milieu réactionnel (environ deux spatules toutes les 10 minutes). Les variations de pH doivent être contrôlées à chaque ajout. La solution devient blanche. Le mélange obtenu est filtré sur Büchner puis le solvant est évaporé à l'évaporateur rotatif et le produit séché sous vide. Poudre blanche, 3.38 g, rdt 93%. Les spectres RMN ¹H / ¹³C et spectrométrie de masse sont en accord avec la structure attendue.

### Exemple 3 : Synthèse du composé 9

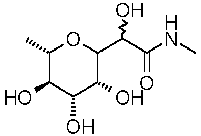

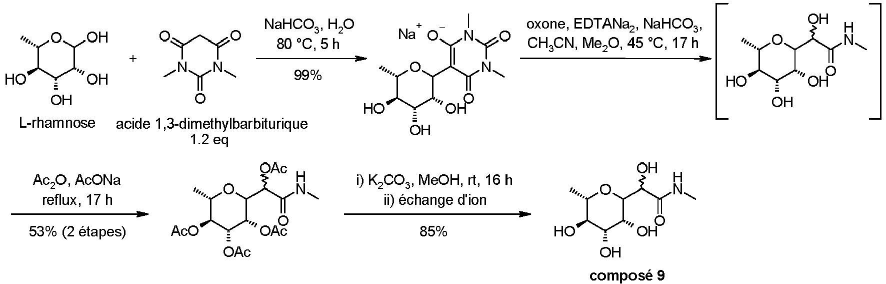

**Etape 1.** Suivant le procédé décrit dans l'exemple 1, l'intermédiaire est obtenu à partir du L-rhamnose (3.4 g, 20.8 mmol). Rdt 99%.

**Etape 2.** Suivant le procédé décrit dans l'exemple 2, le composé **9** brut est obtenu après filtration (5.5 g).

**Etape 3.** Un mélange du composé **9** brut (5.5 g, 20.8 mmol) et NaOAc.3H₂O (4.24 g, 31.2 mmol) dans 100 ml d'Ac₂O (anhydride acétique) est agité à 100 °C pendant 16 h. Après refroidissement, le mélange est versé dans 160 ml d'un bain eau/glace et extrait 3 fois à l'éther diéthylique. Les phases organiques sont rassemblées, lavées trois fois par sat. NaHCO₃ et cinq fois par H₂O. Le solvant est évaporé sous vide, et le résidu obtenu purifié par chromatographie sur silice (20% acétate d'éthyle dans hexane), puis recristallisé de l'éthanol pour donner 4.74 g d'intermédiaire acétylé sous forme de solide blanc, rdt 53% (2 étapes). Les spectres RMN ¹H et spectrométrie de masse sont en accord avec la structure attendue.

**Etape 4.** Un mélange du produit obtenu à l'étape 3 (4.74 g, 11 mmol) et K₂CO₃ (3.04 g, 22 mmol) dans 80 ml de méthanol est agité à t.a. pendant 16 h. Puis de la résine échangeuse d'ions IR-120 (12 g) lavée dans du méthanol absolue est ajoutée. Puis le mélange est agité pendant 30 min, et la résine est éliminée par filtration. Le solvant est évaporé sous vide pour donner 2.2 g de composé **9** pur sous forme d'un solide blanc, rdt 85%. Les spectres RMN ¹H / ¹³C et spectrométrie de masse sont en accord avec la structure attendue.

### Exemple 4 : Synthèse du composé 13

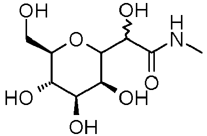

Suivant le procédé décrit dans l'exemple 3, le composé **13** est obtenu à partir du D-mannose (5.85 g, 32.5 mmol). Solide blanc, 2.8 g, rdt étapes 1+2+3 = 68%, rdt étape 4 = 86%. Les spectres RMN ¹H / ¹³C et spectrométrie de masse sont en accord avec la structure attendue.

### Exemple 5 : Synthèse du composé 17

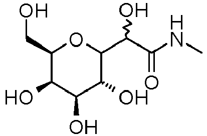

Suivant le procédé décrit dans l'exemple 3, le composé **17** est obtenu à partir du D-galactose (5 g, 27.8 mmol). Solide blanc, 2.4 g, rdt étapes 1+2+3 = 53%, rdt étape 4 = 83%. Les spectres RMN ¹H / ¹³C et spectrométrie de masse sont en accord avec la structure attendue.

### Exemple 6 : Synthèse du composé 21

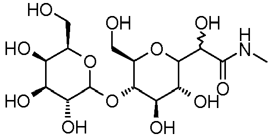

Suivant le procédé décrit dans l'exemple 3, le composé **21** est obtenu à partir du D-lactose (6.2 g, 17 mmol). Solide blanc, 3 g, rdt étapes 1+2+3 = 56%, rdt étape 4 = 83%. Les spectres RMN ¹H / ¹³C et spectrométrie de masse sont en accord avec la structure attendue.

### Exemple 7 : Synthèse du composé 49

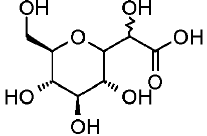

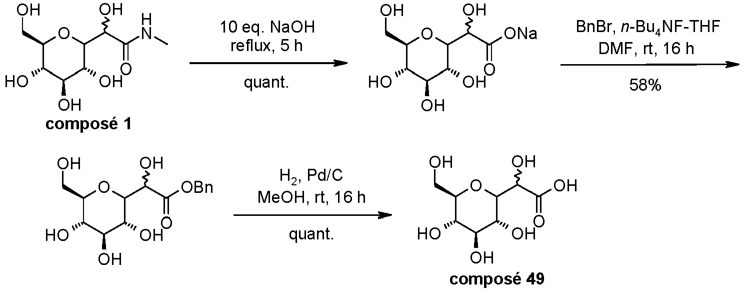

**Etape 1.** A une solution de NaOH (4 g, 100 mmol) dans 50 ml d'eau est ajouté le composé **1** (2.56 g, 10 mmol). Après agitation à 100 °C pendant 5 h, le mélange réactionnel est ajusté à pH ~4 par addition d'une résine échangeuse d'ions. La résine est ensuite éliminée par filtration. Le filtrat est concentré pour obtenir 2.3 g du sel d'acide sous forme d'un solide gris, rdt quant. Les spectres RMN ¹H et spectrométrie de masse sont en accord avec la structure attendue.

**Etape 2.** Un mélange du sel obtenu à l'étape 1 (3.3 g, 12.6 mmol), bromure de benzyle (2.6 g, 15.2 mmol), 12.6 ml de *n*-Bu₄NF en solution 1 M dans le THF, et 20 ml de DMF, est agité à t.a. pendant 16 h. Le mélange est ensuite concentré à sec. Le résidu est purifié par chromatographie sur colonne de silice (dichloromethane / methanol = 10 / 1) pour donner 2.4 g de l'ester de benzyle sous forme d'un solide blanc, rdt 58%. Les spectres RMN ¹H et spectrométrie de masse sont en accord avec la structure attendue. Etape 3. A une solution de l'ester obtenu à l'étape 2 (950 mg, 2.9 mmol) dans 20 ml de méthanol est ajouté 50 mg de Pd/C. Le mélange est agité à t.a. sous H₂ pendant 16 h. Après filtration, le filtrat est concentré à sec pour donner 720 mg de composé **49** sous forme d'un solide blanc, rdt quant. Les spectres RMN ¹H / ¹³C et spectrométrie de masse sont en accord avec la structure attendue.

### Exemple 8 : Synthèse du composé 2

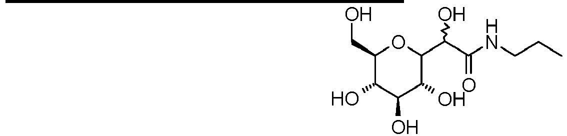

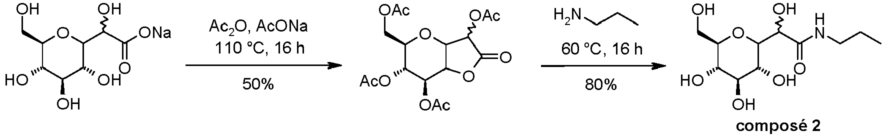

**Etape 1.** Un mélange du sel d'acide décrit dans l'exemple 7 (2.5 g, 10 mmol), AcONa (2 g, 10 mmol), et 20 ml d'Ac₂O est agité à 110 °C pendant 16 h. Puis le mélange est concentré à sec. Le résidu est repris dans 200 ml de NaHCO₃ aq. sat. et extrait 3 fois par acétate d'éthyle. Les phases organiques sont rassemblées, concentrées sous vide, et le résidu obtenu est purifié par chromatographie sur silice (éther de pétrole / acétate d'éthyle = 2/1) pour donner 1.9 g de l'intermédiaire lactone sous forme d'un solide blanc, rdt 50 %. Les spectres RMN ¹H et spectrométrie de masse sont en accord avec la structure attendue.

**Etape 2.** Un mélange de la lactone obtenue à l'étape 1 (1.9 g, 5 mmol) et 10 ml de propylamine est agité à 60 °C pendant 16 h. Le mélange est ensuite concentré à sec. Le résidu obtenu est recristallisé de l'acétate d'éthyle pour donner 1.1 g de composé **2** sous forme d'un solide jaune pâle, rdt 80 %. Les spectres RMN ¹H / ¹³C et spectrométrie de masse sont en accord avec la structure attendue.

### Exemple 9 : Synthèse du composé 3

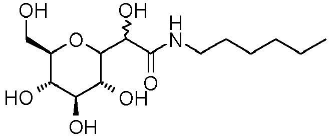

Suivant le procédé décrit dans l'exemple 8, le composé **3** est obtenu à partir de l'intermédiaire lactone (2.5 g, 6 mmol) et 10 ml de n-hexylamine, après 16 h à 100 °C. Recristallisation de l'acétate d'éthyle. Solide jaune pâle, 1.6 g, rdt 83 %. Les spectres RMN ¹H / ¹³C et spectrométrie de masse sont en accord avec la structure attendue.

### Exemple 10 : Synthèse du composé 4

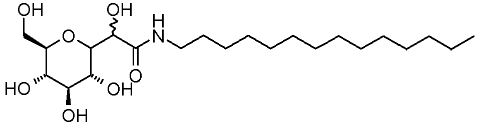

Suivant le procédé décrit dans l'exemple 8, le composé **4** est obtenu à partir de l'intermédiaire lactone (1 g, 2.57 mmol) et de n-tetradecylamine (2.8 g, 12.85 mmol), après 6 h à 150 °C. Recristallisation de l'acétate d'éthyle. Solide jaune pâle, 0.9 g, rdt 81 %. Les spectres RMN ¹H / ¹³C et spectrométrie de masse sont en accord avec la structure attendue.

### Exemple 11 : Activité biologique des composés 1 et 4

Des kératinocytes humains normaux (NHEK) ont été ensemencés et cultivés en milieu de culture pendant 24 heures puis en milieu d'essai pendant 24 heures supplémentaires. Après incubation, le milieu a été remplacé par du milieu d'essai contenant ou non (témoin) les composés à l'essai ou les références puis les cellules ont été incubées pendant 48 heures. Toutes les conditions expérimentales ont été réalisées en n=3. Après incubation, le milieu de culture a été éliminé et les cellules ont été rincées, fixées et perméabilisées. L'expression de GLUT-1 a été déterminée par immunomarquage.

| **Expression de GLUT-1 vs témoin 100%** | |
|---|---|
| **Composé 1 (10 µM)** | **Référence metformine 2 mM** |
| 159% (p<0.05) | 252% (p<0.01) |

| **Expression de GLUT-1 vs témoin 100%** | |
|---|---|
| **Composé 4 (10 µM)** | **Référence metformine 2 mM** |
| 142% (p<0.01) | 296% (p<0.001) |

L'expression de la protéine GLUT-1 est très significativement stimulée par les composés **1** et **4.**

### Exemple 12

On prépare un gel anti-âge pour la peau comprenant (% en poids) :

| | |
|---|---|
| composé de l'exemple 1 | 2% |
| hydroxypropylcellulose (Klucel H de Hercules) | 1% |
| parfum, conservateur | qs |
| isopropanol | 40% |
| eau | qsp 100% |

Une composition similaire est préparée avec le composé de l'exemple 2.

**Exemple 13** : Etudes de l'effet de composés (I) sur la synthèse des glycosaminoglycanes totaux dans des kératinocytes épidermiques humains normaux :
L'étude est faite par mesure de l'incorporation de glucosamine radioactive dans la matrice néosynthétisée par des cultures de kératinocytes épidermiques humains normaux.
L'incorporation de [³H]-glucosamine radioactive indique une néosynthèse spécifique de glycosaminoglycanes totaux.

Le test est effectué sur culture de kératinocytes épidermiques humains normaux en plaque de 96 puits et cultivés en milieu de culture pendant 24 heures :
Milieu de culture : Kératinocytes-SFM complémenté avec Epidermal Growth Factor (EGF) 0,5 ng/ML, extrait Pituitaire 25 µg/ml , Gentamycine 25 µg/ml Conditions de culture : 37 °C ; 5 % CO₂
Le milieu a ensuite été remplacé par du milieu de culture contenant ou non (témoin) le composé (I) testé ou les références le 4-Nitrophényl-B-D-xylopyranoside (testé à 300 µM) et le 4-méthylumbelliferyl-B-D-xylopyranoside (testé à 54 µM) ou le contrôle solvant (DMSO à 0,1 %) puis les cellules ont été incubées pendant 72 heures avec ajout du marqueur [³H]-glucosamine pendant les 24 dernières heures d'incubation.

Le test a été effectué avec 3 essais pour chaque composé testé, aux concentrations de 1, 10, 100 et 300 µM.

A la fin de l'incubation, les glycosamioglycanes ont été extraits des cellules avec un tampon chaotropique. Le taux de glucosamine radioactive incorporée est mesuré en fin de test par adsorption des molécules anioniques sur billes de Q-sepharose suivit d'une désorption des molécules peu et moyennement anioniques avec urée 6M plus NaCl 0,2M. Une fois lavée, le comptage de la radioactivité incorporée dans les molécules très cationiques restées sur le support est effectué.

Les résultats ont été évalués par rapport à un témoin constitué par des cellules n'ayant pas été traitées avec le composé (I).

Un témoin positif (TGFβ à 0,25 ng/ml) connu pour stimuler la synthèse des GAGs a été introduit dans le test comme référence positive.

Les résultats sont présentés dans le tableau suivant :

| Traitement | | Valeur moyenne | sd | n | % | P |
|---|---|---|---|---|---|---|
| Témoin | | 15109 | 892 | 3-3 | 100 | -- |
| 4-nitrophenyl β-D-xylopyranoside | | 31280 | 544 | 3 | 207 | < 0,001 |
| 4-methylumbelliferyl-β-D-xylopyranoside | | 32202 | 670 | | 213 | < 0,001 |
| Contrôle solvant (DMSO 0,1 %) | | 17863 | 932 | 3-3 | 118 | > 0,05 |
| Composé 1 | 1 µM | 21591 | 293 | 3 | 125 | 0,01<p<0,05 |
| | 10 µM | 20216 | 709 | 3 | 117 | p>0.05 |
| | 100µM | 20451 | 898 | 3 | 118 | p>0.05 |
| | 300µM | 16850 | 2070 | 3 | 97 | p>0.05 |
| Composé 2 | 1 µM | 14638 | 838 | 3 | 110 | p>0.05 |
| | 10 µM | 16779 | 1032 | 3 | 126 | 0,01<p<0,05 |
| | 100µM | 16435 | 2319 | 3 | 123 | p>0.05 |
| | 300µM | 16929 | 1865 | 3 | 127 | p>0.05 |
| Composé 5 | 1 µM | 17792 | 1030 | 3 | 134 | 0,01<p<0,05 |
| | 10 µM | 16133 | 1949 | 3 | 121 | p>0.05 |
| | 100µM | 15835 | 2289 | 3 | 119 | p>0.05 |
| | 300µM | 15495 | 2171 | 3 | 116 | p>0.05 |
| Composé 9 | 1 µM | 16162 | 2008 | 3 | 120 | p>0.05 |
| | 10 µM | 15439 | 985 | 3 | 114 | p>0.05 |
| | 100µM | 17655 | 2224 | 3 | 131 | p>0.05 |
| | 300µM | 22845 | 400 | 3 | 169 | < **0,001** |
| Composé 13 | 1 µM | 16090 | 1523 | 3 | 119 | p>0.05 |
| | 10 µM | 17340 | 598 | 3 | 129 | 0,001<p<0,01 |
| | 100µM | 18139 | 2075 | 3 | 134 | p>0.05 |
| | 300µM | 18804 | 301 | 3 | 139 | p>0.05 |
| Composé 17 | 1 µM | 17387 | 815 | 3 | 129 | 0.01<p<0,05 |
| | 10 µM | 16614 | 699 | 3 | 123 | 0.01<p<0,05 |
| | 100µM | 17861 | 945 | 3 | 132 | 0.01<p<0,05 |
| | 300µM | 14498 | 1047 | 3 | 108 | p>0.05 |
| Composé 21 | 1 µM | 18416 | 1507 | 3 | 142 | 0.01<p<0,05 |
| | 10 µM | 17611 | 717 | 3 | 136 | 0.01<p<0,05 |
| | 100µM | 17796 | 1682 | 3 | 137 | p>0.05 |
| | 300µM | 17429 | 665 | 3 | 134 | 0.01<p<0,05 |

| | | | | | | |
|---|---|---|---|---|---|---|
| Les valeurs mesurées sont données en coups par minute (cpm) sd : déviation standard p : intervalle de confiance n : replicates | | | | | | |

Ces résultats montrent que :
- les composés 1 et 5 stimulent l'incorporation de glucosamine radioactive à la concentration de 1 µM ;
- les composés 2 et 13 stimulent l'incorporation de glucosamine radioactive à la concentration de 10 µM ;
- le composé 9 stimule l'incorporation de glucosamine radioactive à la concentration de 300 µM ;
- le composé 17 stimule l'incorporation de glucosamine radioactive aux concentrations de 1 et 10 et 100 µM ;
- le composé 21 stimule l'incorporation de glucosamine radioactive aux concentrations de 1 et 10 et 300 µM .

Ainsi, les composés (I) testés stimulent l'incorporation de glucosamine radioactive ce qui indique une néosynthèse de glycosaminoglycanes.

## Revendications

1. Procédé de traitement des matières kératiniques notamment de la peau, en particulier pour traiter les signes cutanés du vieillissement, comprenant l'application sur lesdites matières d'au moins une composition cosmétique comprenant un ou plusieurs composés de formule **(I)** suivante 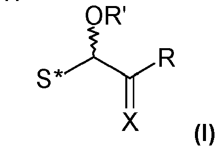
ainsi que leurs solvates tels que les hydrates, leurs isomères optiques, tautomères et leurs sels de base ou d'acide, organiques ou minéraux ;
formule **(I)** dans laquelle :
- **S*** désigne un monosaccharide choisi parmi le D-glucose, le D-xylose, le L-rhamnose, le D-mannose, le D-galactose ou un disaccharide choisi parmi D-lactose, D-maltose et D-cellobiose ledit radical mono ou disaccharide étant relié au reste de la molécule par une liaison entre l'atome de carbone C₁ d'un des sucres dudit radical mono ou disaccharide, cette liaison pouvant être anomérique α ou β ;
- **X** représente un atome d'oxygène ;
- **R'** représente un atome d'hydrogène ;
- **R** représente un groupe choisi parmi : i) hydroxy ; et ii) amino -NR₁R₂ avec R₁ et R₂, identiques ou différents, représentant un atome d'hydrogène, un groupe (C₁-C₁₈)alkyle, de préférence alkyle en C₁-C₁₆ tel que méthyle, n-propyle, n-hexyle, n-tétradécyle (n-C₁₄).

2. Procédé selon la revendication précédente dans lequel le ou les composé(s) de formule **(I)** comporte(nt) un radical sucre **S*** qui représente un sucre choisi parmi le D-glucose, le L-xylose et le D-lactose, plus particulièrement, **S*** désigne le D-glucose ou le D-xylose ; plus préférentiellement, **S*** désigne le D-glucose.

3. Procédé selon une quelconque des revendications précédentes dans lequel le ou les composé(s) de formule **(I)** comporte(nt) un radical **R** qui représente un groupe amino -NR₁R₂ avec R₁ et R₂, identiques ou différents, représentent un atome d'hydrogène, (C₁-C₁₄)alkyle, linéaire ou ramifié, tel que méthyle, n-propyle, n-hexyle, n-tétradécane (n-C₁₄) ; de préférence le composés (I) est de formule (la) : 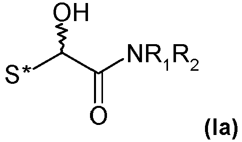
ainsi que ses solvates tels que les hydrates, ses isomères optiques, tautomères et ses sels de bases ou d'acide, organiques ou minéraux,
Formule **(Ia)** dans laquelle **R₁**,et **R₂** sont tels que définis précédemment, **S*** est tel que défini dans une quelconque des revendications 1 ou 2.

4. Procédé selon une quelconque des revendications 1 ou 2 dans lequel le ou les composé(s) de formule **(I)** comporte(nt) un radical **R** qui représente un groupe hydroxy, de préférence le composé **(I)** est de formule **(Ic)** : 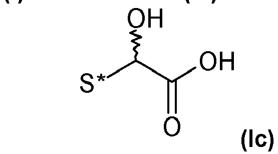 ainsi que leurs solvates tels que les hydrates, leurs isomères optiques, géométriques, tautomères et leurs sels de bases ou d'acide, organiques ou minéraux, avec **S*** est tel que défini dans une quelconque des revendications 1 ou 2.

5. Procédé selon une quelconque des revendications précédentes dans lequel le ou les composé(s) de formule **(I)** est(sont) choisis parmi les composés suivants :
| | |
|---|---|
| **1** | 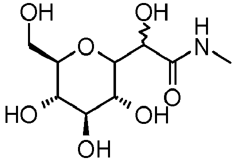 |
| **2** | 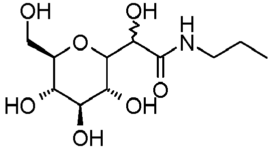 |
| **3** | 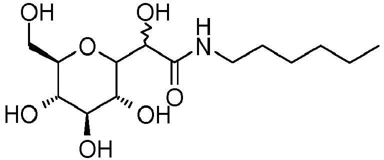 |
| **4** | 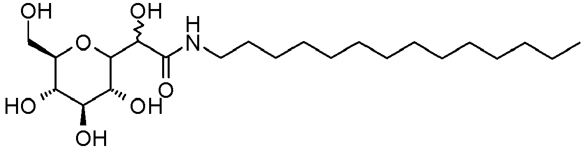 |
| **5** | 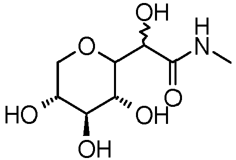 |
| **6** | 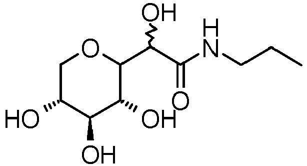 |
| **7** | 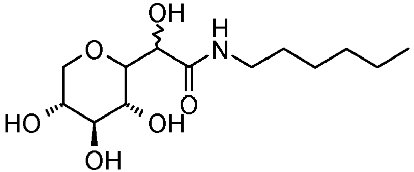 |
| **8** | 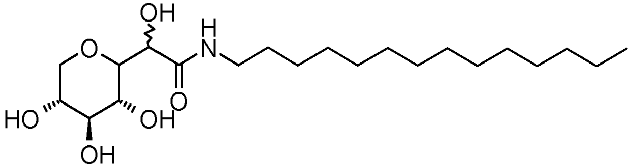 |
| **9** | 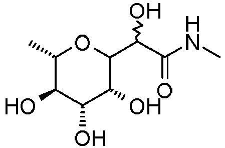 |
| **10** | 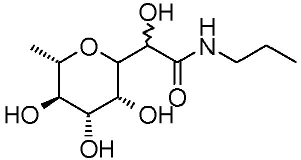 |
| **11** | 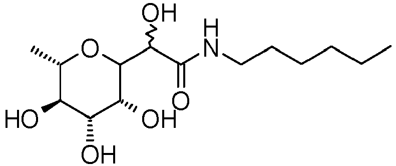 |
| **12** | 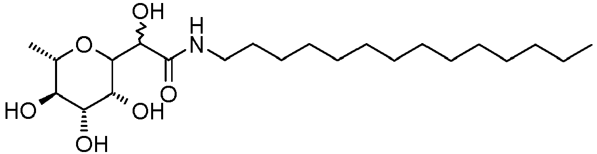 |
| **13** | 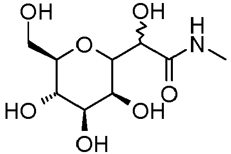 |
| **14** | 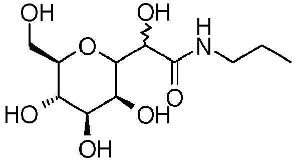 |
| **15** | 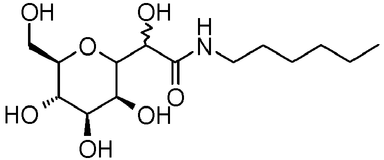 |
| **16** | 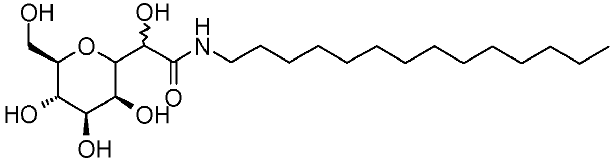 |
| **17** | 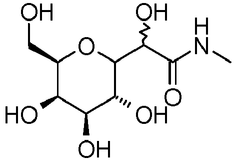 |
| **18** | 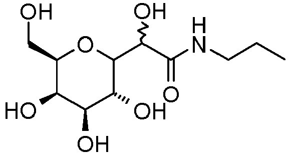 |
| **19** | 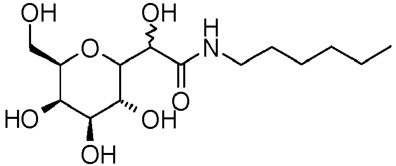 |
| **20** | 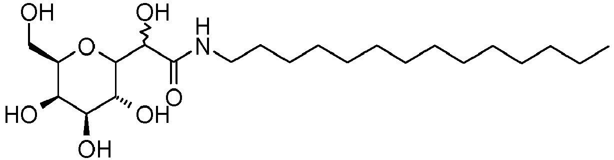 |
| **21** | 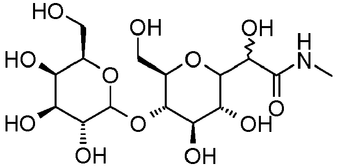 |
| **22** | 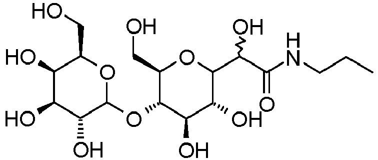 |
| **23** | 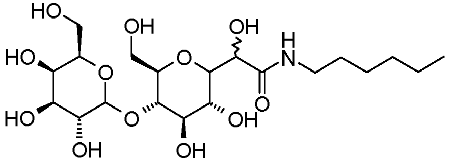 |
| **24** | 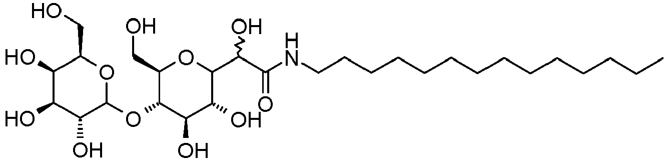 |
| **39** | 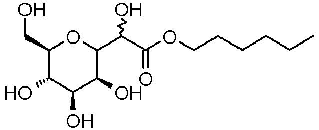 |
| **49** | 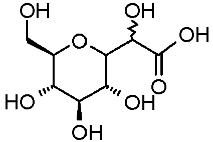 |
| **50** | 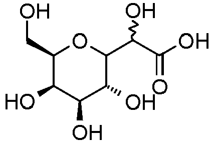 |
| **51** | 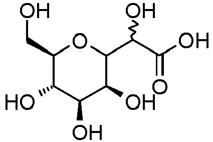 |
| **52** | 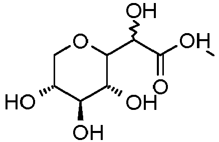 |
| **53** | 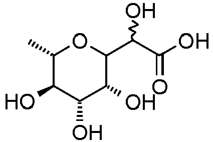 |
| **54** | 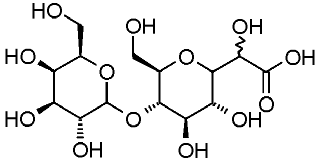 |
ainsi que leurs solvates tels que les hydrates, et leurs sels de base ou d'acide, organiques ou minéraux.

6. Composition cosmétique comprenant au moins un composé de formule **(I)** tel que défini dans une quelconque des revendications précédentes, de préférence au moins un composé de formule **(I)** choisi parmi **(Ia),** et **(Ic),** tels que définis dans les revendications 3 ou 4 et composés **1** à **39** et **49** à **54** tels que définis dans la revendication précédente, de préférence étant entendu que le ou les composés de formule (I) est(sont) tel(s) que **S*** représente un mannose, **R'** représente un atome d'hydrogène, alors **R** est différent d'un groupe diéthylamino N(Et)₂.

7. Composé de formule **(I)** 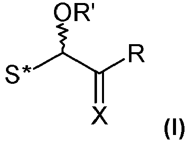 dans laquelle :
- **S*** représente un radical sucre monosaccharide choisi parmi : le glucose, le galactose, le mannose, le xylose, le rhamnose, ou un disaccharide choisi parmi : le lactose, le maltulose ; ledit radical **S*** comprenant un ou plusieurs radicaux **-ORₛ** et éventuellement un ou deux radicaux -NHR'ₛ,
ledit radical mono ou disaccharide étant relié au reste de la molécule par une liaison entre l'atome de carbone C¹ du sucre ou d'un des sucres et cette liaison pouvant être anomérique α ou β;
- **X** représente un atome d'oxygène ;
- **R'** représente : i) un atome d'hydrogène ;;
- **R** représente un groupe choisi parmi :
i) hydroxy ;
ii) amino NR₁R₂ avec R₁ et R₂ désignant indépendamment un atome d'hydrogène, un radical (C₁-C₁₆)alkyle tel que méthyle, n-propyle, n-hexyle, n-tetradecyle,
**Rₛ** représente un atome d'hydrogène,
lorsque **S*** représente un mannose, **R'** représente un atome d'hydrogène, alors **R** est différent d'un groupe diéthylamino N(Et)₂.

8. Composés selon la revendication 7, choisi parmi :
| | |
|---|---|
| **1** | 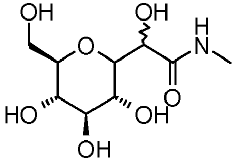 |
| **2** | 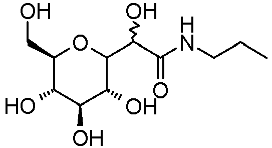 |
| **3** | 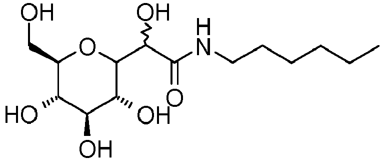 |
| **4** | 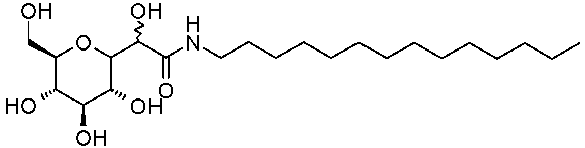 |
| **5** | 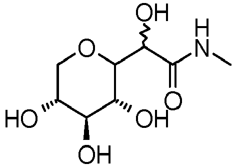 |
| **6** | 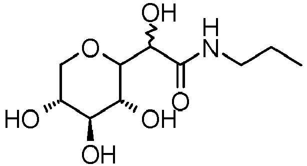 |
| **7** | 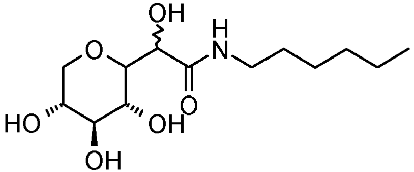 |
| **8** | 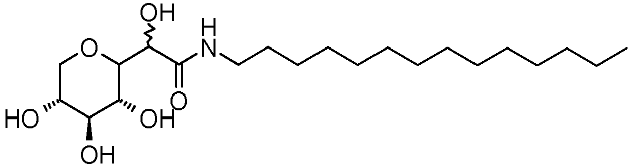 |
| **9** | 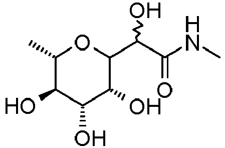 |
| **10** | 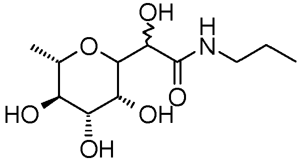 |
| **11** | 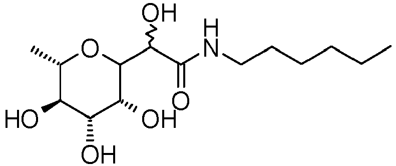 |
| **12** | 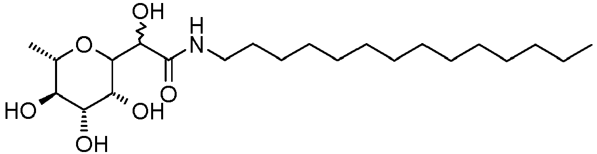 |
| **13** | 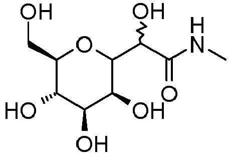 |
| **14** | 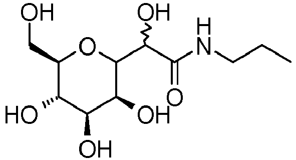 |
| **15** | 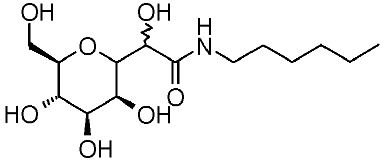 |
| **16** | 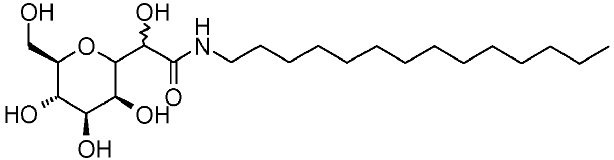 |
| **17** | 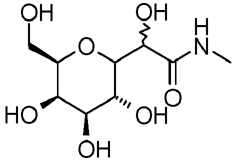 |
| **18** | 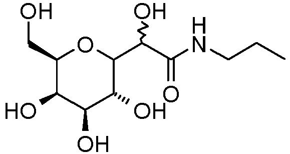 |
| **19** | 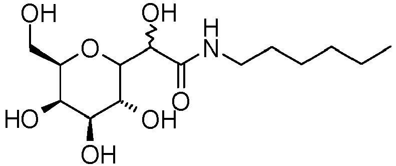 |
| **20** | 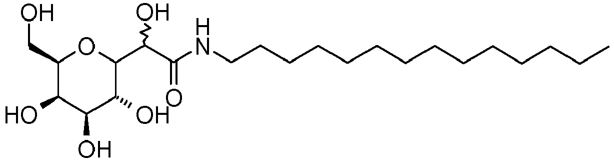 |
| **21** | 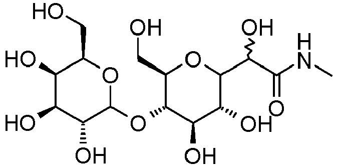 |
| **22** | 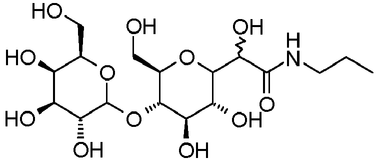 |
| **23** | 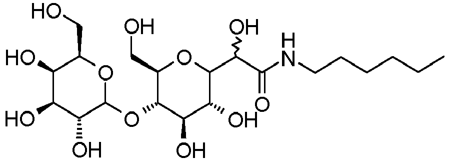 |
| **24** | 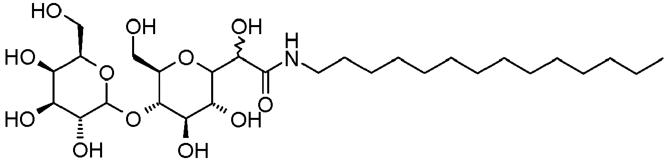 |
| **49** | 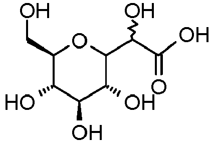 |
| **50** | 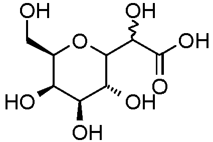 |
| **51** | 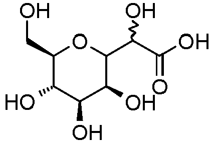 |
| **52** | 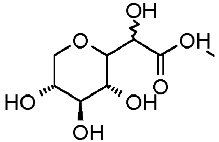 |
| **53** | 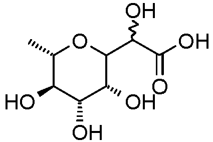 |
| **54** | 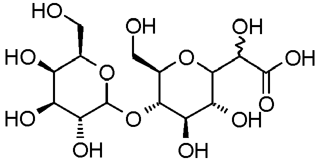 |
ainsi que leurs solvates tels que les hydrates, et leurs sels de base ou d'acide, organiques ou minéraux.

9. Composés selon la revendication 7 ou 8, **caractérisé en ce qu'**ils sont choisis parmi les composés pour lesquels R = -NR₁R₂ ;

10. Composés selon l'une des revendications 7 8 ou 9, choisi parmi :
| | |
|---|---|
| **1** | 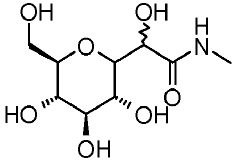 |
| **2** | 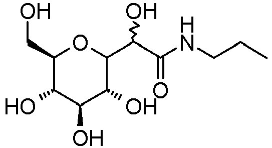 |
| | |
|---|---|
| **5** | 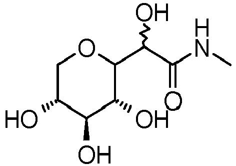 |
| **6** | 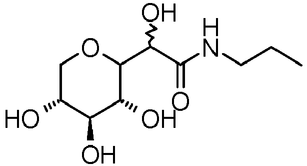 |
| | |
|---|---|
| **9** | 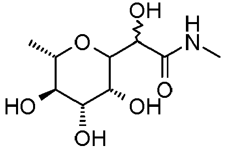 |
| **10** | 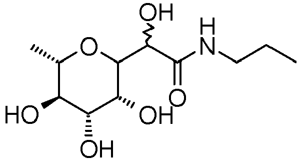 |
| **13** | 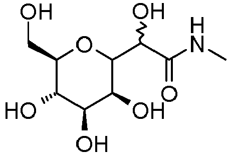 |
| | |
|---|---|
| **14** | 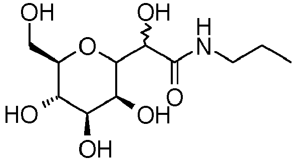 |
| **17** | 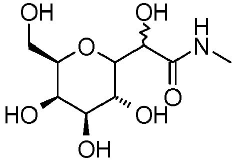 |
| **18** | 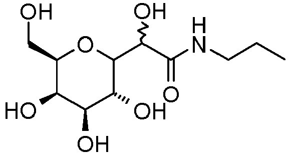 |
| **21** | 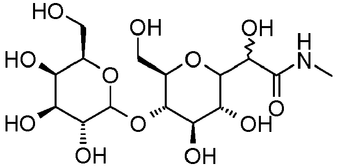 |
| **22** | 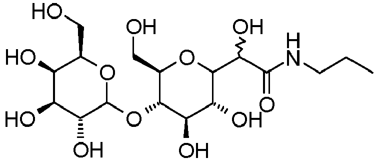 |
ainsi que leurs solvates tels que les hydrates, et leurs sels de base ou d'acide, organiques ou minéraux,

11. Utilisation non thérapeutique, de composé **(I)** tel que défini dans une quelconque des revendications 1 à 5, de préférence au moins un composé de formule **(I)** choisi parmi **(Ia), (Ic),** tels que définis dans les revendications 3 ou 4 et composés **1** à **39** et **49** à **54** tels que définis dans la revendication 5 ; pour diminuer et/ou retarder les signes du vieillissement de la peau et/ou de ses annexes.

## Patentansprüche

1. Verfahren zur Behandlung von Keratinmaterialien, insbesondere der Haut, insbesondere zur Behandlung von Hautalterungsanzeichen, umfassend das Aufbringen mindestens einer kosmetischen Zusammensetzung, die eine oder mehrere Verbindungen der nachstehenden Formel **(I)** 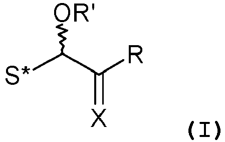
sowie die Solvate davon wie Hydrate, die optischen Isomere und Tautomere davon und die organischen oder mineralischen Basen- oder Säuresalze davon umfasst, auf die Materialien;
wobei in Formel **(I):**
- **S*** ein Monosaccharid, das aus D-Glucose, D-Xylose, L-Rhamnose, D-Mannose und D-Galactose ausgewählt ist, oder ein Disaccharid, das aus D-Lactose, D-Maltose und D-Cellobiose ausgewählt ist, bedeutet, wobei der Mono- oder Disaccharidrest über eine Bindung zwischen dem C₁-Kohlenstoffatom eines der Zucker des Mono- bzw. Disaccharidrests an den Rest des Moleküls gebunden ist, wobei diese Bindung α- or β-anomer sein kann;
- **X** für ein Sauerstoffatom steht;
- **R**' für ein Wasserstoffatom steht;
- **R** für eine Gruppe steht, die aus i) Hydroxyl und ii) Amino -NR₁R₂ ausgewählt ist, wobei R₁ und R₂, die gleich oder verschieden sein können, für ein Wasserstoffatom oder eine (C₁-C₁₈)Alkylgruppe, vorzugsweise C₁-C₁₆-Alkylgruppe wie Methyl, n-Propyl, n-Hexyl, n-Tetradecyl (n-C₁₄), stehen.

2. Verfahren nach dem vorhergehenden Anspruch, wobei die Verbindung bzw. die Verbindungen der Formel **(I)** einen Zuckerrest **S*** enthält bzw. enthalten, der für einen Zucker, der aus D-Glucose, L-Xylose und D-Lactose ausgewählt ist, steht; spezieller **S*** D-Glucose oder D-Xylose bezeichnet; weiter bevorzugt **S*** D-Glucose bedeutet.

3. Verfahren nach einem der vorhergehenden Ansprüche, wobei die Verbindung bzw. die Verbindungen der Formel **(I)** einen Rest **R** enthält bzw. enthalten, der für eine Aminogruppe -NR₁R₂ steht, wobei R₂ und R₂, die gleich oder verschieden sein können, für ein Wasserstoffatom, (C₁-C₁₄)Alkyl, geradkettig oder verzweigt, wie Methyl, n-Propyl, n-Hexyl, n-Tetradecan (n-C₁₄), stehen; vorzugsweise die Verbindung (I) die Formel (Ia) aufweist: 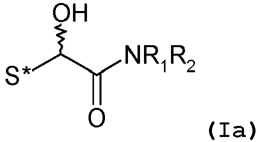
sowie die Solvate davon wie Hydrate, die optischen Isomere und Tautomere davon und die organischen oder mineralischen Basen- oder Säuresalze davon,
wobei in Formel (**Ia**) **R₁** und **R₂** wie oben definiert sind und **S*** wie in Anspruch 1 oder 2 definiert ist.

4. Verfahren nach Anspruch 1 oder 2, wobei die Verbindung bzw. die Verbindungen der Formel **(I)** einen Rest **R** enthält bzw. enthalten, der für eine Hydroxygruppe steht, vorzugsweise die Verbindung **(I)** die Formel **(Ic)** aufweist: 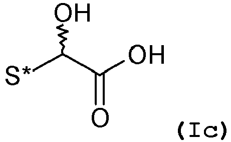 sowie die Solvate davon wie Hydrate, die optischen und geometrischen Isomere und Tautomere davon und die organischen oder mineralischen Basen- oder Säuresalze davon, wobei **S*** wie in Anspruch 1 oder 2 definiert ist.

5. Verfahren nach einem der vorhergehenden Ansprüche, wobei die Verbindung bzw. die Verbindungen der Formel **(I)** aus den folgenden Verbindungen ausgewählt ist bzw. sind:
| | |
|---|---|
| **1** | 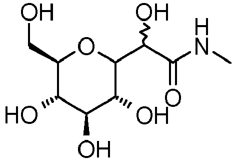 |
| **2** | 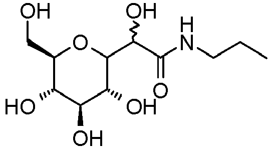 |
| **3** | 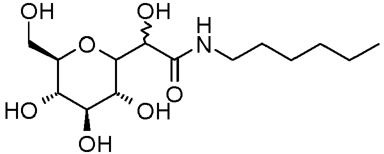 |
| **4** | 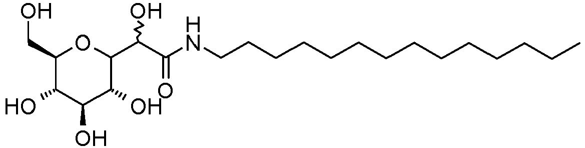 |
| **5** | 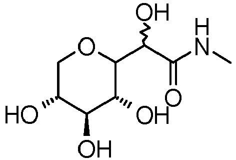 |
| **6** | 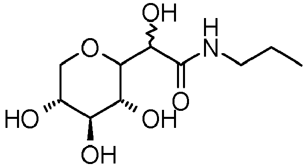 |
| **7** | 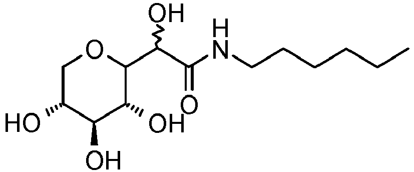 |
| **8** | 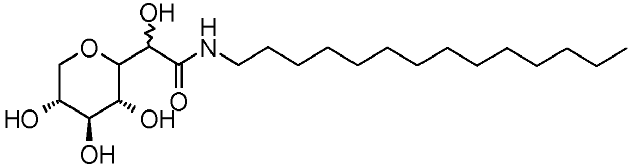 |
| **9** | 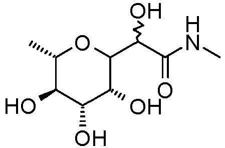 |
| **10** | 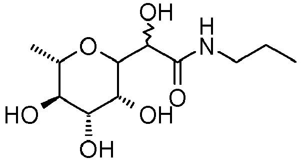 |
| **11** | 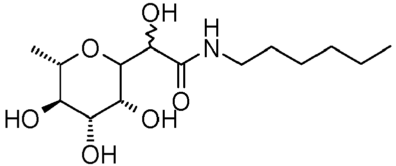 |
| **12** | 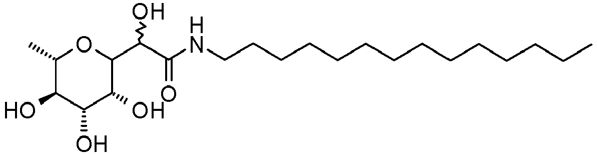 |
| **13** | 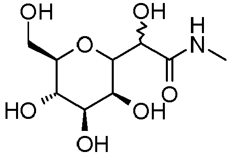 |
| **14** | 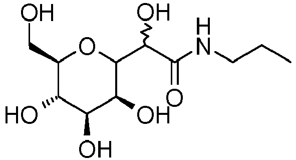 |
| **15** | 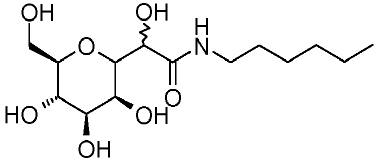 |
| **16** | 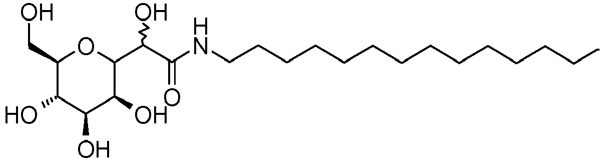 |
| **17** | 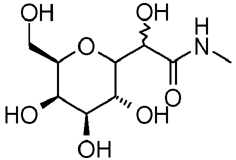 |
| **18** | 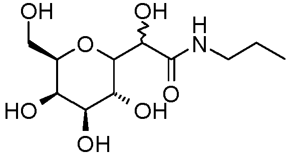 |
| **19** | 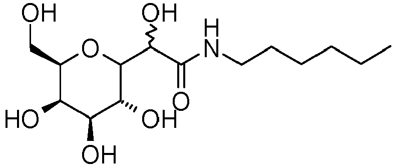 |
| **20** | 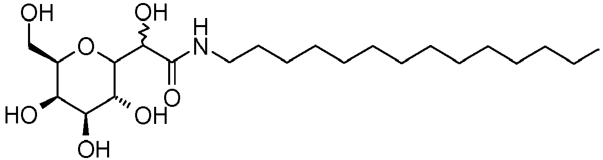 |
| **21** | 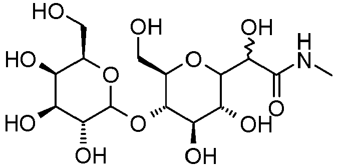 |
| **22** | 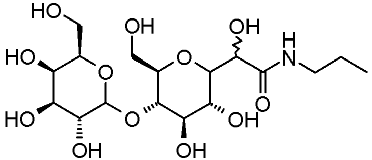 |
| **23** | 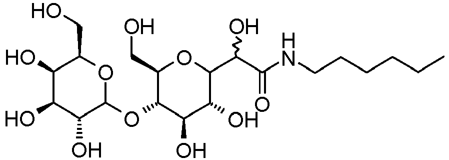 |
| **24** | 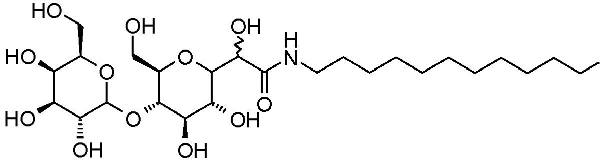 |
| **39** | 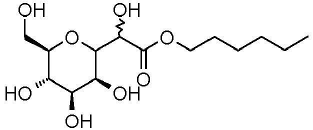 |
| **49** | 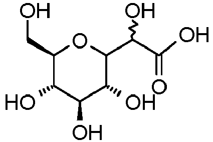 |
| **50** | 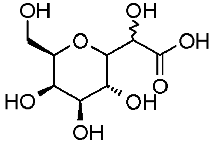 |
| **51** | 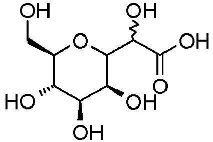 |
| **52** | 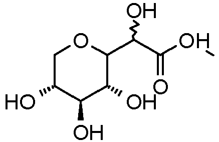 |
| **53** | 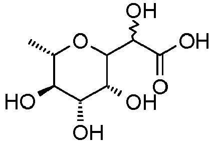 |
| **54** | 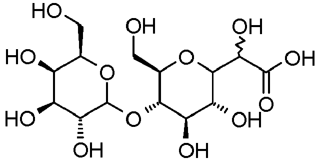 |
sowie die Solvate davon wie Hydrate, und die organischen oder mineralischen Basen- oder Säuresalze davon.

6. Kosmetische Zusammensetzung, umfassend mindestens eine Verbindung der Formel **(I)** gemäß einem der vorhergehenden Ansprüche, vorzugsweise mindestens eine Verbindung der Formel **(I),** die aus **(Ia)** und **(Ic)** gemäß Anspruch 3 oder 4 und den Verbindungen **1** bis **39** und **49** bis **54** gemäß dem vorhergehenden Anspruch ausgewählt ist, wobei es sich vorzugsweise versteht, dass die Verbindung bzw. die Verbindungen der Formel (I) so beschaffen ist bzw. sind, dass **S*** für eine Mannose steht, **R'** für ein Wasserstoffatom steht und **R** von einer Diethylaminogruppe N(Et)₂ verschieden ist.

7. Verbindung der Formel **(I)** 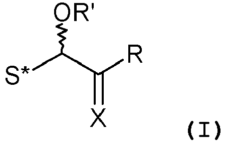 in der:
- **S*** für einen Monosaccharid-Zuckerrest, der aus Glucose, Galactose, Mannose, Xylose und Rhamnose ausgewählt ist, oder ein Disaccharid, das aus Lactose und Maltulose ausgewählt ist, steht; wobei der Rest **S*** einen oder mehrere Reste **-ORₛ** und gegebenenfalls einen oder zwei Reste -NHR'ₛ umfasst,
wobei der Mono- oder Disaccharidrest über eine Bindung zwischen dem C¹-Kohlenstoffatom des Zuckers bzw. eines der Zucker an den Rest des Moleküls gebunden ist, wobei diese Bindung α- or β-anomer sein kann;
- **X** für ein Sauerstoffatom steht;
- **R'** für i) ein Wasserstoffatom steht;
- **R** für eine Gruppe steht, die aus
i) Hydroxyl;
ii) Amino NR₁R₂, wobei R₂ und R₂ unabhängig ein Wasserstoffatom oder einen (C₁-C₁₆)Alkylrest wie Methyl, n-Propyl, n-Hexyl oder n-Tetradecyl bedeuten, ausgewählt ist;
**Rₛ** für ein Wasserstoffatom steht,
dann, wenn **S*** für eine Mannose steht und **R'** für ein Wasserstoffatom steht, **R** von einer Diethylaminogruppe N(Et)₂ verschieden ist.

8. Verbindungen nach Anspruch 7, ausgewählt aus:
| | |
|---|---|
| **1** | 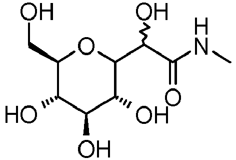 |
| **2** | 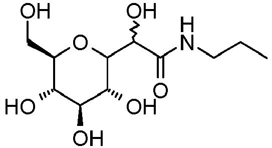 |
| **3** | 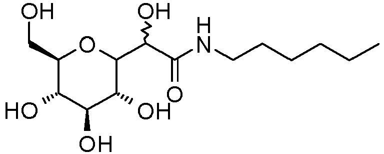 |
| **4** | 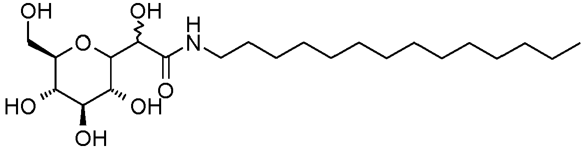 |
| **5** | 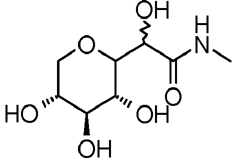 |
| **6** | 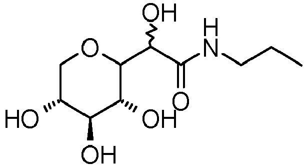 |
| **7** | 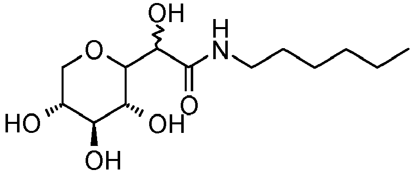 |
| **8** | 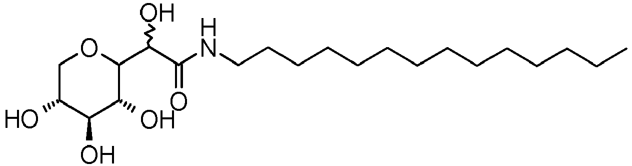 |
| **9** | 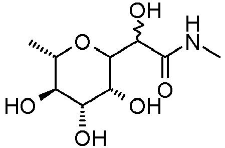 |
| **10** | 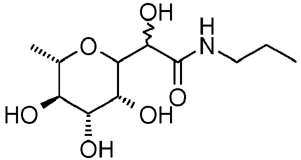 |
| **11** | 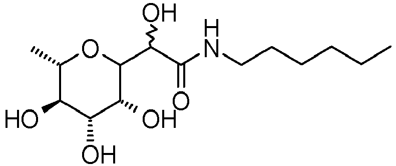 |
| **12** | 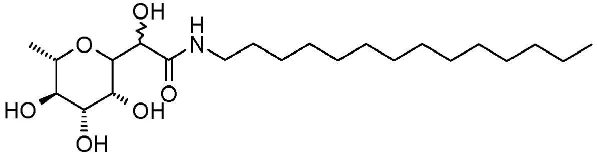 |
| **13** | 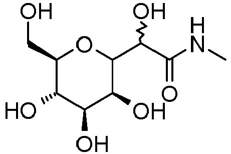 |
| **14** | 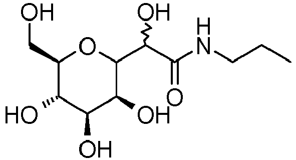 |
| **15** | 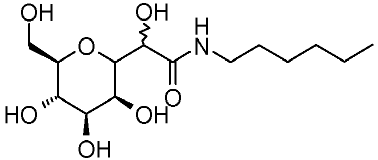 |
| **16** | 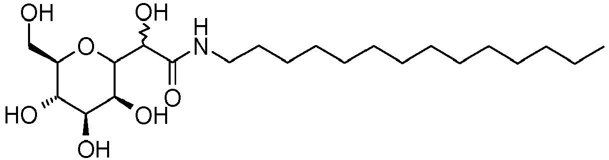 |
| **17** | 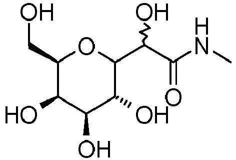 |
| **18** | 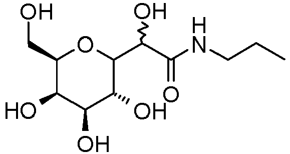 |
| **19** | 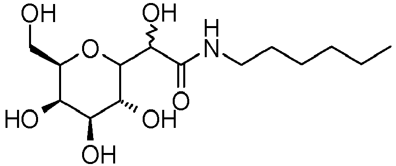 |
| **20** | 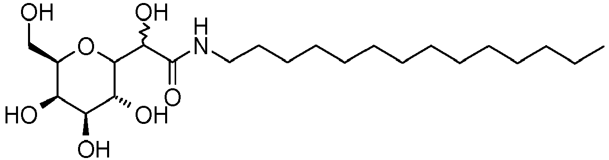 |
| **21** | 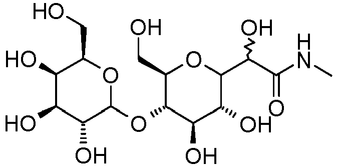 |
| **22** | 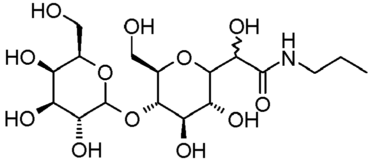 |
| **23** | 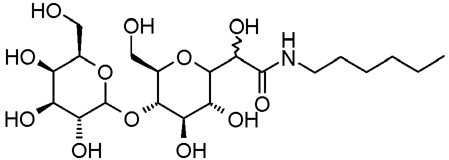 |
| 24 | 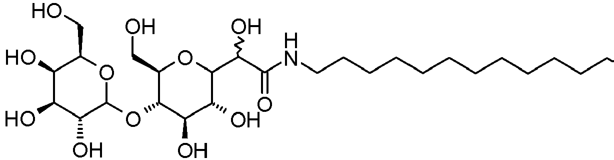 |
| 49 | 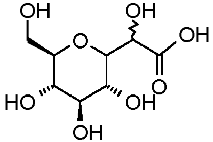 |
| 50 | 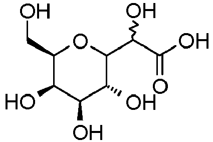 |
| 51 | 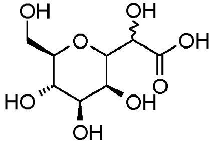 |
| 52 | 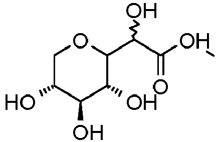 |
| 53 | 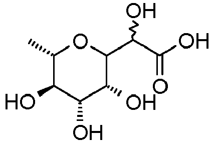 |
| 54 | 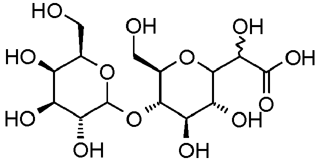 |
sowie die Solvate davon wie Hydrate, und die organischen oder mineralischen Basen- oder Säuresalze davon.

9. Verbindungen nach Anspruch 7 oder 8, **dadurch gekennzeichnet, dass** sie aus den Verbindungen ausgewählt sind, für die R = -NR₁R₂.

10. Verbindungen nach einem der Ansprüche 7, 8 oder 9, ausgewählt aus:
| | |
|---|---|
| 1 | 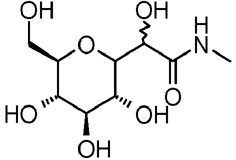 |
| 2 | 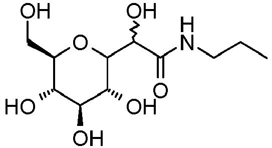 |
| | |
|---|---|
| 5 | 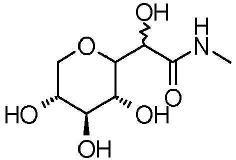 |
| 6 | 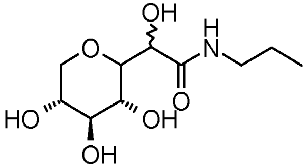 |
| | |
|---|---|
| 9 | 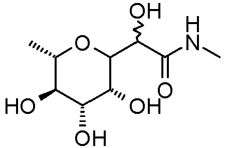 |
| 10 | 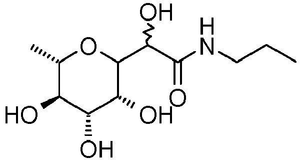 |
| 13 | 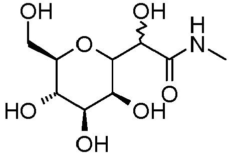 |
| 14 | 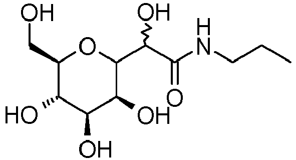 |
| | |
|---|---|
| 17 | 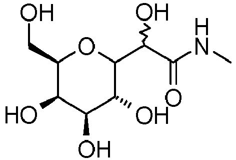 |
| 18 | 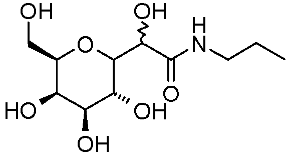 |
| 21 | 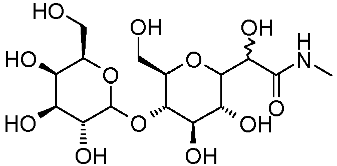 |
| 22 | 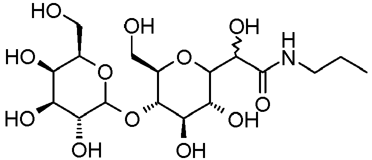 |
sowie die Solvate davon wie Hydrate, und die organischen oder mineralischen Basen- oder Säuresalze davon.

11. Nichttherapeutische Verwendung von Verbindung **(I)** gemäß einem der Ansprüche 1 bis 5, vorzugsweise mindestens einer Verbindung der Formel **(I),** die aus **(Ia)** und **(Ic)** gemäß Anspruch 3 oder 4 und den Verbindungen **1** bis **39** und **49** bis **54** gemäß Anspruch 5 ausgewählt ist; zur Verringerung und/oder Verzögerung der Anzeichen der Alterung der Haut und/oder der Hautanhangsgebilde.

## Claims

1. Process for treating keratin materials, in particular the skin, in particular for treating the signs of skin aging, comprising the application to said materials of at least one cosmetic composition comprising one or more compounds of formula **(I)** below 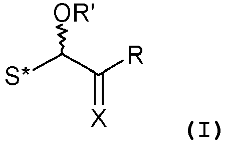
and also the solvates thereof such as hydrates, the optical and geometric isomers and tautomers thereof and the organic or mineral base or acid salts thereof;
in which formula **(I):**
- **S*** denotes a monosaccharide chosen from D-glucose, D-xylose, L-rhamnose, D-mannose and D-galactose or a disaccharide chosen from D-lactose, D-maltose and D-cellobiose, said monosaccharide or disaccharide radical being connected to the rest of the molecule by a bond between the C₁ carbon atom of one of the sugars of said monosaccharide or disaccharide radical, this bond possibly being α or β anomeric;
- **X** represents an oxygen atom;
- **R'** represents a hydrogen atom;
- **R** represents a group chosen from: i) hydroxyl; and ii) amino - NR₁R₂ with R₁ and R₂, which may be identical or different, representing a hydrogen atom, a (C₁-C₁₈)alkyl group, preferably C₁-C₁₆ alkyl group such as methyl, n-propyl, n-hexyl, n-tetradecyl (n-C₁₄).

2. Process according to the preceding claim, wherein the compound(s) of formula **(I)** comprise(s) a sugar radical **S*** which represents a sugar chosen from D-glucose, L-xylose and D-lactose; more particularly, **S*** denotes D-glucose or D-xylose; more preferentially, S* denotes D-glucose.

3. Process according to either one of the preceding claims, wherein the compound(s) of formula **(I)** comprise(s) a radical **R** which represents an amino group - NR₁R₂ with R₁ and R₂, which may be identical or different, representing a hydrogen atom, linear or branched C₁-C₁₄ alkyl such as methyl, n-propyl, n-hexyl, or n-tetradecane (n-C₁₄); preferably, the compound (I) is of formula (Ia): 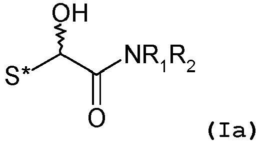
and also the solvates thereof such as hydrates, the optical isomers and tautomers thereof and the organic or mineral base or acid salts thereof,
in which formula **(Ia)**, **R₁** and **R₂** are as defined above, **S*** is as defined in either one of Claims 1 and 2.

4. Process according to either one of Claims 1 and 2, wherein the compound(s) of formula **(I)** comprise(s) a radical **R** which represents a hydroxyl group, preferably the compound **(I)** is of formula **(Ic):** 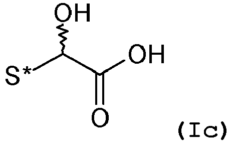 and also the solvates thereof such as hydrates, the optical and geometric isomers and tautomers thereof and the organic or mineral base or acid salts thereof, with **S*** being as defined in either one of Claims 1 and 2.

5. Process according to any one of the preceding claims, wherein the compound(s) of formula **(I)** is (are) chosen from the following compounds:
| | |
|---|---|
| 1 | 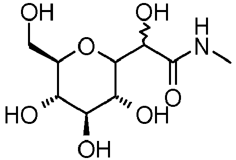 |
| 2 | 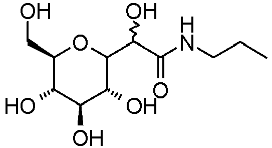 |
| 3 | 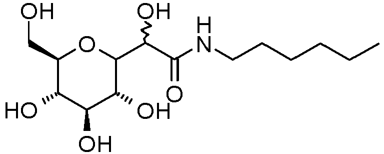 |
| 4 | 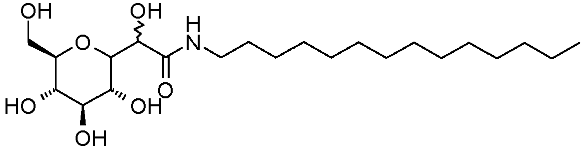 |
| 5 | 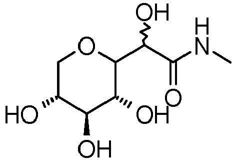 |
| 6 | 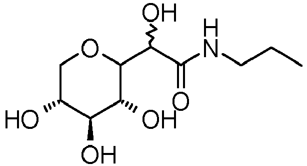 |
| 7 | 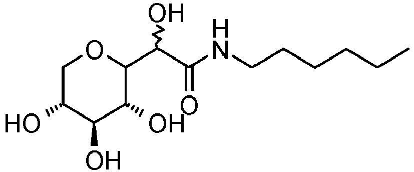 |
| 8 | 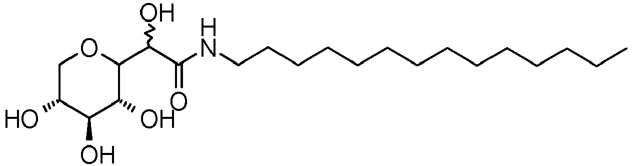 |
| 9 | 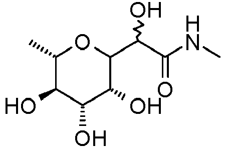 |
| 10 | 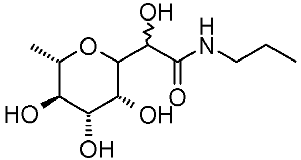 |
| 11 | 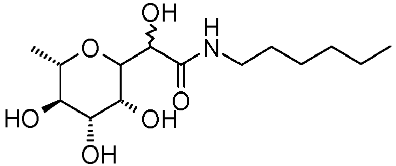 |
| 12 | 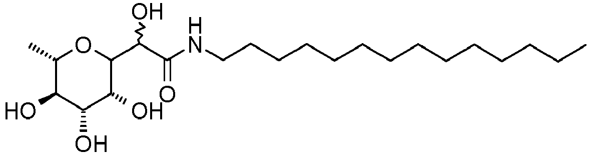 |
| 13 | 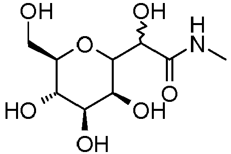 |
| 14 | 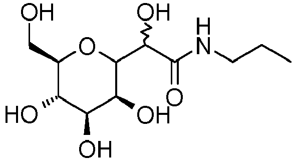 |
| 15 | 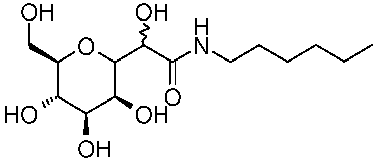 |
| 16 | 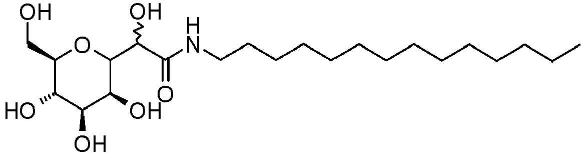 |
| 11 | 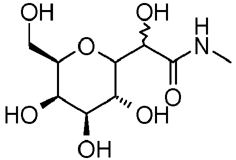 |
| 18 | 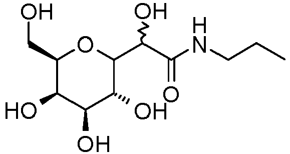 |
| 19 | 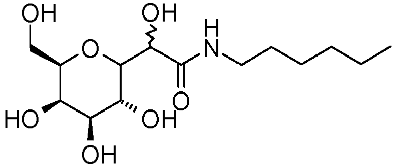 |
| 20 | 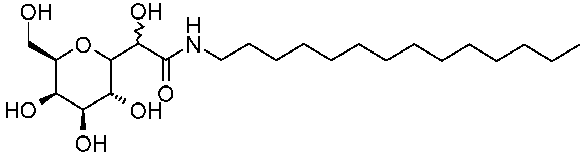 |
| 21 | 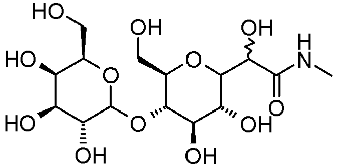 |
| 22 | 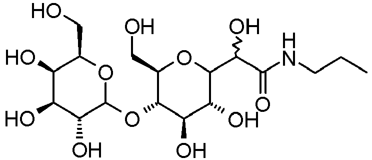 |
| 23 | 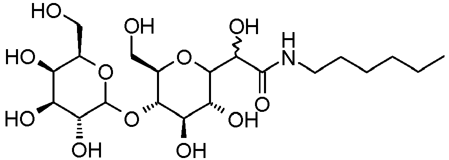 |
| 24 | 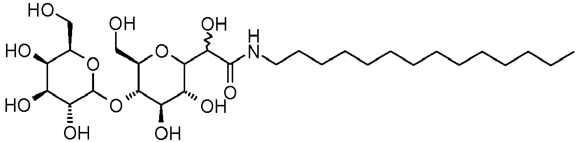 |
| 39 | 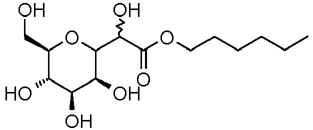 |
| 49 | 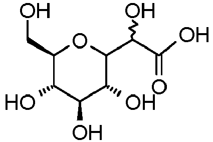 |
| 50 | 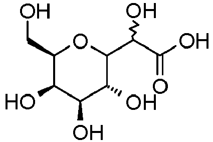 |
| 51 | 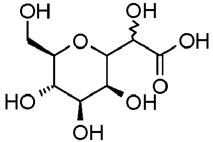 |
| 52 | 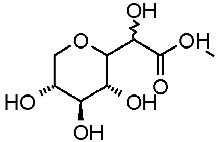 |
| 53 | 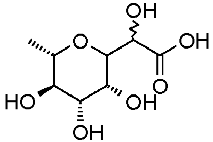 |
| 54 | 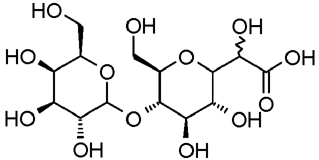 |
and also the solvates thereof such as hydrates, and the organic or mineral base or acid salts thereof.

6. Cosmetic composition comprising at least one compound of formula **(I)** as defined in any one of the preceding claims, preferably at least one compound of formula **(I)** chosen from **(Ia)** and **(Ic)** as defined in Claims 3 or 4 and compounds **1** to **39** and **49** to **54** as defined in the preceding claim, it preferably being understood that the compound(s) of formula (I) is (are) such that S* represents a mannose and **R'** represents a hydrogen atom, then **R** is other than a diethylamino group N(Et)₂.

7. Compound of formula **(I)** 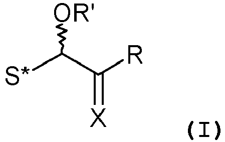 wherein:
- **S*** represents a monosaccharide sugar radical chosen from: glucose, galactose, mannose, xylose and rhamnose, or a disaccharide chosen from: lactose and maltulose; said radical **S*** comprising one or more radicals **-ORₛ** and optionally one or two radicals -NHR'ₛ,
said monosaccharide or disaccharide radical being connected to the rest of the molecule by a bond between the C¹ carbon atom of the sugar or one of the sugars and this bond possibly being α or β anomeric;
- **X** represents an oxygen atom;
- **R'** represents: i) a hydrogen atom;
- **R** represents a group chosen from:
i) hydroxyl;
ii) amino NR₁R₂ with R₁ and R₂ independently denoting a hydrogen atom, a (C₁-C₁₆)alkyl radical such as methyl, n-propyl, n-hexyl or n-tetradecyl,
**Rₛ** represents a hydrogen atom,
when **S*** represents a mannose and **R'** represents a hydrogen atom, then **R** is other than a diethylamino group N(Et)₂.

8. Compounds according to Claim 7, chosen from:
| | |
|---|---|
| 1 | 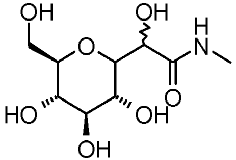 |
| 2 | 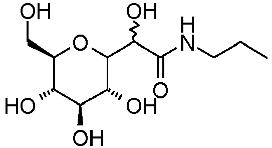 |
| 3 | 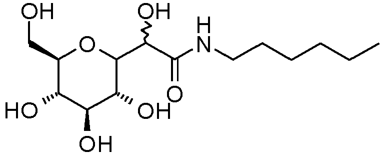 |
| 4 | 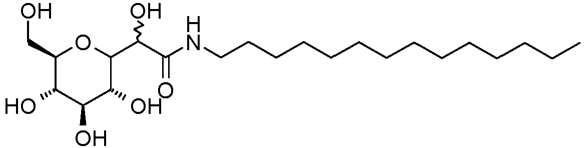 |
| 5 | 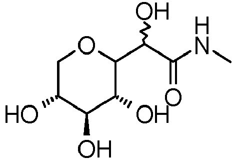 |
| 6 | 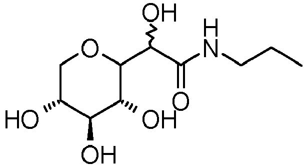 |
| 7 | 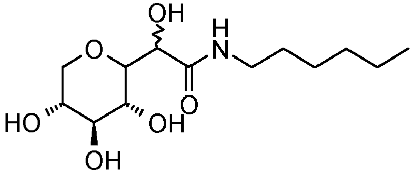 |
| 8 | 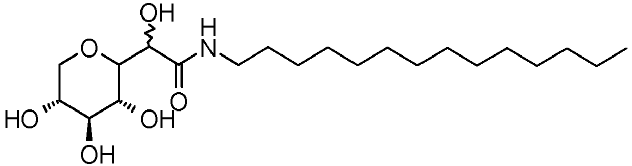 |
| 9 | 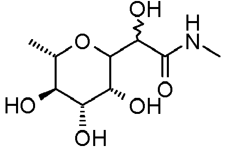 |
| 10 | 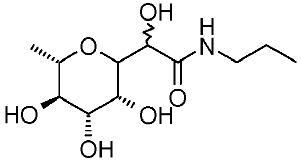 |
| 11 | 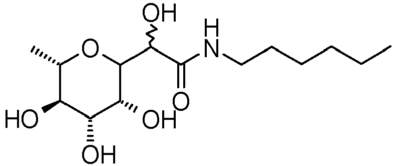 |
| 12 | 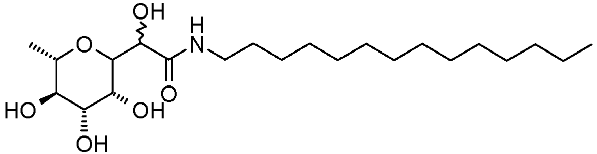 |
| 13 | 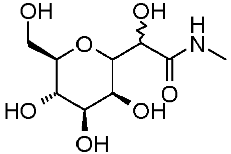 |
| 14 | 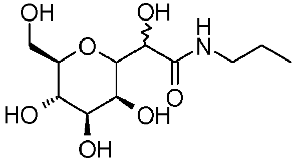 |
| 15 | 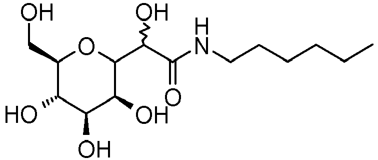 |
| 16 | 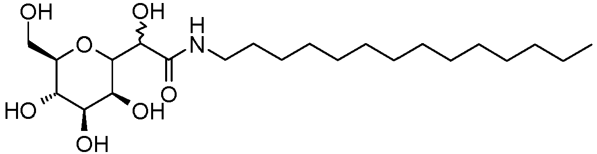 |
| 17 | 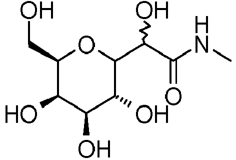 |
| 18 | 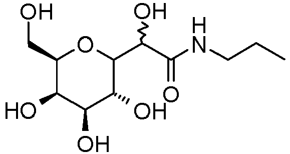 |
| 19 | 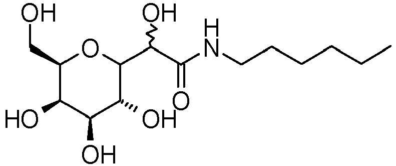 |
| 20 | 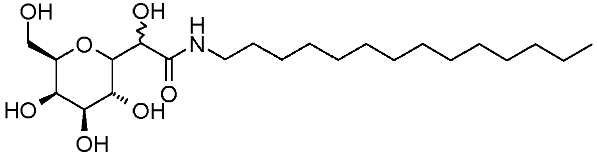 |
| 21 | 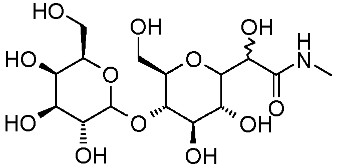 |
| 22 | 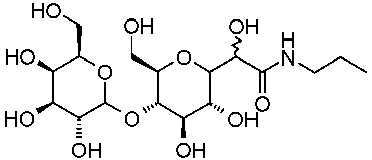 |
| 23 | 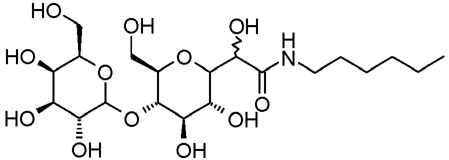 |
| 24 | 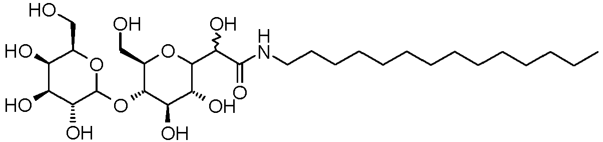 |
| 49 | 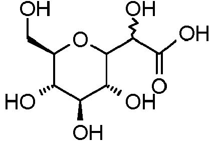 |
| 50 | 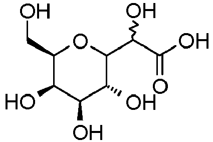 |
| 51 | 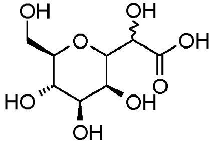 |
| 52 | 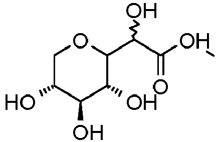 |
| 53 | 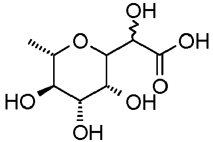 |
| 54 | 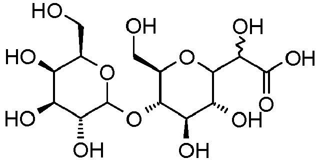 |
and also the solvates thereof such as hydrates, and the organic or mineral base or acid salts thereof.

9. Compounds according to Claim 7 or 8, **characterized in that** they are chosen from the compounds for which R = -NR1R2;

10. Compounds according to one of Claims 7, 8 or 9, chosen from:
| | |
|---|---|
| 1 | 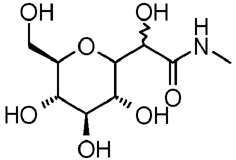 |
| 2 | 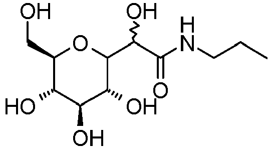 |
| 5 | 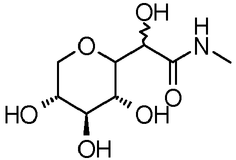 |
| 6 | 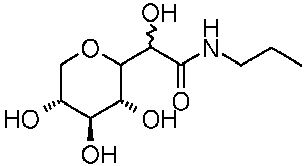 |
| 9 | 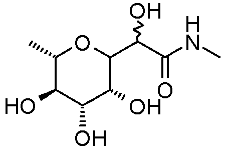 |
| 10 | 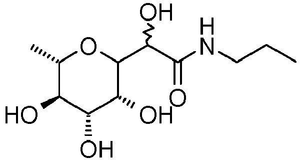 |
| 13 | 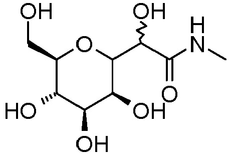 |
| 14 | 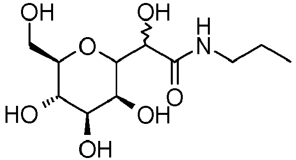 |
| 17 | 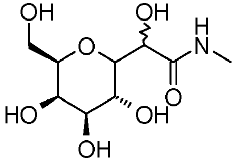 |
| 18 | 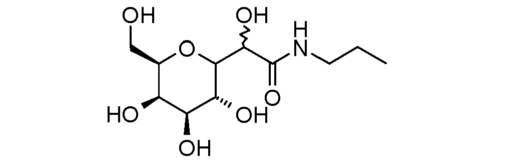 |
| 21 | 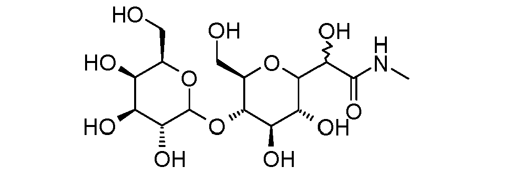 |
| 22 | 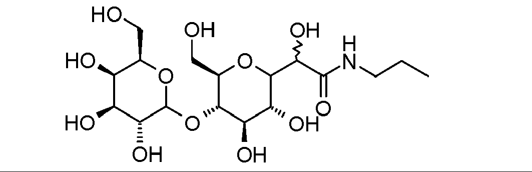 |
and also the solvates thereof such as hydrates, and the organic or mineral base or acid salts thereof.

11. Non-therapeutic use, of compound **(I)** as defined in any one of Claims 1 to 5, preferably at least one compound of formula **(I)** chosen from **(Ia)** and **(Ic),** as defined in Claims 3 or 4 and compounds **1** to **39** and **49** to **54** as defined in Claim 5; for decreasing and/or delaying the signs of aging of the skin and/or skin appendages.
